(19) Europäisches Patentamt
European Patent Office
Office européen des brevets

(11) EP 1 604 648 A1

(12) # DEMANDE DE BREVET EUROPEEN

(43) Date de publication:
**14.12.2005 Bulletin 2005/50**

(51) Int Cl.[7]: **A61K 7/48**

(21) Numéro de dépôt: **05291072.6**

(22) Date de dépôt: **18.05.2005**

(84) Etats contractants désignés:
**AT BE BG CH CY CZ DE DK EE ES FI FR GB GR HU IE IS IT LI LT LU MC NL PL PT RO SE SI SK TR**
Etats d'extension désignés:
**AL BA HR LV MK YU**

(30) Priorité: **11.06.2004 FR 0406370**

(71) Demandeur: **L'OREAL**
**75008 Paris (FR)**

(72) Inventeur: **Toumi, Béatrice**
**91370 Verrieres le Buisson (FR)**

(74) Mandataire: **Boulard, Denis**
**L'OREAL - D.I.P.I.**
**25-29 Quai Aulagnier**
**92600 Asnières (FR)**

(54) **Composition cosmétique comprenant un polymère éthylénique greffé**

(57) L'invention a pour objet une composition cosmétique de maquillage ou de soin de la peau comprenant une dispersion de particules d'un polymère éthylénique greffé dans une phase grasse liquide, la composition étant apte à former un dépôt ayant un module complexe E* inférieur ou égal à 200 MPa.

La composition permet d'obtenir un dépôt sur la peau ayant une bonne adhérence et/ou confortable et/ou ayant une bonne résistance au transfert, notamment en présence de sébum.

Application au maquillage et au soin de la peau.

EP 1 604 648 A1

## Description

La présente invention a pour objet une composition cosmétique comprenant un polymère particulier destinée à être appliquée sur la peau d'êtres humains.

La composition selon l'invention peut être une composition de maquillage ou une composition de soin de la peau, et de préférence une composition de maquillage.

La composition de maquillage peut être un fond de teint, un fard à paupières, un fard à joue, un produit anticernes, un eye-liner, un produit de maquillage du corps. Plus spécialement, l'invention porte sur une composition de fond de teint.

La composition de soin peut être un produit de soin de la peau du corps et du visage, notamment un produit solaire, un produit de coloration de la peau (tel qu'un autobronzant).

Les compositions de fond de teint sont couramment employées pour apporter une couleur esthétique à la peau, notamment au visage. Ces produits de maquillage contiennent généralement des huiles, des pigments et/ou charges et éventuellement des additifs comme des actifs cosmétiques ou dermatologiques.

Ces compositions, lorsqu'elles sont appliquées sur la peau, présentent l'inconvénient de transférer, c'est-à-dire de se déposer au moins en partie, en laissant des traces, sur certains supports avec lesquels elles peuvent être mises en contact et notamment un verre, une tasse, une cigarette, un vêtement ou la peau. Il s'ensuit une tenue médiocre du film appliqué, nécessitant de renouveler régulièrement l'application de la composition de fond de teint ou de rouge à lèvres. Par ailleurs, l'apparition de ces traces inacceptables notamment sur les cols de chemisier peut écarter certaines femmes de l'utilisation de ce type de maquillage.

De plus, le sébum excrété par la peau au cours du temps modifie également les propriétés du maquillage. En particulier, le sébum ne favorise pas l'adhésion du maquillage sur la peau et le transfert du maquillage est encore plus important, engendrant une perte notable du maquillage restant sur la peau.

On recherche donc des compositions de maquillage de la peau « sans transfert » qui présentent l'avantage de former un dépôt résistant au transfert, en particulier en présence de sébum, notamment qui ne se dépose pas, au moins en partie, sur les supports avec lesquels elles sont mises en contact (verre, vêtements, cigarette, tissus).

Pour améliorer la tenue des produits de maquillage, il est connu d'utiliser des polymères filmogènes. Par exemple, les documents US-6074654 et WO02/067877 proposent d'employer des résines de silicones.

Toutefois, certains polymères intéressants pour l'obtention de propriétés de non transfert ne sont pas toujours bien adaptés pour être appliqués sur la peau. En effet, la peau du visage est soumise à de nombreux mouvements et il est nécessaire que le film de maquillage déposé puisse supporter ces contraintes sans se fissurer ou se craqueler, voire aussi sans se détacher de la peau. Or tous les polymères filmogènes ne permettent pas d'obtenir un dépôt satisfaisant sur la peau, notamment lorsqu'ils sont présents en une teneur élevée dans le dépôt obtenu après le séchage complet de la composition appliquée sur la peau. Le polymère présente alors une mauvaise adhérence sur la peau engendrant une mauvaise tenue du maquillage. En outre, le polymère provoque des tiraillements sur la peau, rendant ainsi le maquillage inconfortable pour l'utilisatrice.

La présente invention a donc pour but de fournir une nouvelle voie de formulation d'un produit cosmétique qui permette d'obtenir un dépôt, notamment un maquillage, sur la peau présentant de bonnes propriétés d'adhérence, de cohésion sous contraintes mécaniques et de confort, ainsi que de bonnes propriétés de non transfert, notamment en présence de sébum.

Les inventeurs ont découvert qu'il est possible d'obtenir une telle composition en utilisant un polymère particulier.

De façon plus précise, la présente invention a donc pour objet une composition cosmétique de maquillage ou de soin de la peau comprenant une dispersion de particules, de préférence solides, d'un polymère éthylénique greffé dans une phase grasse liquide telle que décrite ci-après, la composition étant notamment telle que définie ci-après.

Avantageusement, le polymère éthylénique greffé est tel que, lorsqu'il est présent en quantité suffisante dans la composition, cette dernière est apte à former un dépôt ayant un module complexe E* inférieur ou égal à 200 MPa.

L'invention a également pour objet un procédé de maquillage de la peau, comprenant l'application sur les matières kératiniques, notamment sur la peau d'une composition telle que définie précédemment.

L'invention a aussi pour objet l'utilisation d'une composition telle que définie précédemment pour obtenir un dépôt, notamment un maquillage sur la peau ayant une bonne adhérence et/ou confortable et/ou ayant une bonne résistance au transfert, notamment en présence de sébum.

L'invention a encore pour objet l'utilisation, dans une composition cosmétique d'une dispersion de particules (de préférence solides) d'un polymère éthylénique greffé en dispersion dans une phase grasse liquide, la composition étant notamment apte à former un dépôt ayant un module complexe E* inférieur ou égal à 200 MPa, pour obtenir un dépôt, notamment un maquillage, sur la peau ayant une bonne adhérence et/ou confortable et/ou ayant une bonne résistance au transfert, notamment en présence de sébum.

Avantageusement, le polymère éthylénique greffé utilisé dans la composition selon l'invention est tel que lorsqu'il est présent en quantité suffisante dans la composition, cette dernière est apte à former un dépôt ayant un module complexe E* inférieur ou égal à 200 MPa, notamment allant de 0,1 MPa à 200 MPa.

Le module complexe E* du dépôt obtenu avec la composition selon l'invention est déterminé selon le protocole de mesure décrit ci-après.

Méthode de mesure du module complexe E* d'un film

On applique sur une plaque de téflon une quantité de la composition pour obtenir après séchage pendant 7 jours sur une plaque thermostatée à 35 °C un dépôt d'environ 100 à 150 μm d'épaisseur. On découpe ensuite dans le film sec un échantillon de 10 mm de largeur et de 15 mm de long.

Avec cet échantillon, on effectue des essais de viscoélasticimétrie avec un appareil de spectrométrie dynamique de type DMTA (Dynamical and Mechanical Temperature Analysis ou Analyse dynamique et mécanique en température), par exemple le modèle DMA2980 de la société TA Instruments.

On impose à l'échantillon une sollicitation de traction. L'échantillon subit une force statique, par exemple de 0,01 N, à laquelle se superpose un déplacement sinusoïdal de, par exemple, ± 8 μm à la fréquence de 1 Hz. On travaille ainsi dans le domaine linéaire, sous de faibles niveaux de déformation. Cette sollicitation est effectuée sur l'échantillon à des températures variant de -50 °C à + 100 °C, avec une variation de température de 3 °C par minute. Préférentiellement, on maintient l'échantillon pendant 5 minutes à une isotherme de - 50 °C.
On mesure alors le module complexe E* = E' + iE" du film testé en fonction de la température.
De ces mesures, on déduit les modules dynamiques E' de conservation (appelé aussi module d'Young) et E" de perte, ainsi que le pouvoir amortissant : tgδ = E"/E'.

Par module complexe E* du dépôt (film), on entend la norme du nombre complexe

$$E^* = \sqrt{(E'^2 + E''^2)}.$$

Avantageusement, la composition selon l'invention est apte à former un dépôt, notamment un film, ayant un module complexe E* de conservation inférieur ou égal à 150 MPa, notamment allant de 0,1 MPa à 150 MPa, de préférence inférieur
ou égal à 100 MPa, notamment allant de 0,1 MPa à 100 MPa, et préférentiellement inférieur ou égal à 50 MPa, notamment allant de 1 MPa à 50 MPa.

De préférence, la composition selon l'invention est apte à former un dépôt, notamment un film, ayant un pouvoir amortissant tgδ supérieur ou égal à 0,7 , notamment allant de 0,7 à 2, préférentiellement supérieur ou égal à 0,9, notamment allant de 0,9 à 2, et plus préférentiellement supérieur ou égal à 1,1 , notamment allant de 1,1 à 2. Le pouvoir amortissant du dépôt est déterminé selon le protocole décrit précédemment.

Avantageusement, la composition selon l'invention est apte à former un dépôt ayant au moins une température de transition vitreuse comprise entre 0 °C et 40 °C.

La mesure de la température de transition du dépôt (ou film) obtenu avec la composition selon l'invention est effectuée en traçant la courbe des valeurs de tgδ, obtenues lors des essais de viscoélasticimétrie effectués comme indiqués précédemment , en fonction de la température. La (ou les) température(s) de transition vitreuse Tg du polymère corresponde(nt) à la (aux) température(s) à la (au)quelle(s) on détecte un maximum local sur cette courbe.

La composition cosmétique selon l'invention comprend une dispersion de particules, de préférence solides, d'un polymère éthylénique greffé dans une phase grasse liquide.

La composition cosmétique selon l'invention est une composition compatible avec les matières kératiniques, notamment la peau.

Par polymère "éthylénique", on entend un polymère obtenu par polymérisation de monomères comprenant une insaturation éthylénique.

La dispersion de polymère éthylénique greffé est notamment exempte de polymère stabilisant distinct dudit polymère greffé, tels que ceux décrits dans EP749747, et les particules de polymère éthylénique greffé ne sont donc pas stabilisées en surface par de tels polymères stabilisants additionnels. Le polymère greffé est donc dispersé dans la phase grasse liquide en l'absence de stabilisant additionnel en surface des particules du polymère greffé.

Par polymère greffé, on entend un polymère ayant un squelette comprenant au moins une chaîne latérale pendante ou située en bout de chaîne, et de préférence pendante.

Avantageusement, le polymère éthylénique greffé comprend un squelette éthylénique insoluble dans ladite phase grasse liquide, et des chaînes latérales liées de manière covalente audit squelette et solubles dans ledit milieu de dispersion.

Le polymère éthylénique greffé est notamment un polymère non réticulé. En particulier, le polymère est obtenu

par polymérisation de monomères comprenant un seul groupement polymérisable.

De préférence, le polymère éthylénique greffé est un polymère filmogène.

Par polymère "filmogène", on entend un polymère apte à former à lui seul ou en présence d'un agent auxiliaire de filmification, un film continu et adhérent sur un support, notamment sur les matières kératiniques.

Selon un mode de réalisation de l'invention, le polymère éthylénique greffé est un polymère acrylique greffé.

Le polymère éthylénique greffé est notamment susceptible d'être obtenu par polymérisation radicalaire dans un milieu organique de polymérisation :

- d'au moins un monomère éthylénique, en particulier d'au moins un monomère acrylique et éventuellement d'au moins un monomère additionnel vinylique non acrylique, pour former ledit squelette insoluble ; et
- d'au moins un macromonomère comportant un groupe terminal polymérisable pour former les chaînes latérales, ledit macromonomère ayant une masse moléculaire moyenne en poids supérieure ou égale à 200 et la teneur en macromonomère polymérisé représentant de 0,05 à 20 % en poids du polymère.

La phase grasse liquide peut contenir le milieu organique de polymérisation.

Le milieu organique liquide de dispersion, correspondant au milieu dans lequel est fourni le polymère greffé, peut être identique au milieu de polymérisation.

Toutefois, le milieu de polymérisation peut être substitué en tout ou partie par un autre milieu organique liquide. Cet autre milieu organique liquide peut être ajouté, après polymérisation, au milieu de polymérisation. Ce dernier est ensuite évaporé en tout ou partie.

La phase grasse liquide peut contenir des composés liquides organiques autres que ceux présents dans le milieu de dispersion. Ces autres composés sont choisis de manière à ce que le polymère greffé reste à l'état de dispersion dans la phase grasse liquide.

Le milieu liquide organique de dispersion est présent dans la phase grasse liquide de la composition selon l'invention du fait de l'introduction dans la composition de la dispersion de polymère greffé obtenue.

La phase grasse liquide comprend, de préférence majoritairement un ou plusieurs composés organiques liquides (ou huiles) tels que définis ci-après.

En particulier, la phase grasse liquide est une phase organique liquide non aqueuse et non miscible à l'eau à la température ambiante (25 °C).

On entend par "composé organique liquide" un composé non aqueux qui est à l'état liquide à la température ambiante (25 °C) et qui s'écoule donc de son propre poids.

On entend par "composé siliconé" un composé contenant au moins un atome de silicium.

Parmi les composés organiques liquides ou huiles, notamment volatiles ou non volatiles, pouvant être présents dans le milieu organique liquide de dispersion, on peut citer :

- les composés organiques liquides, notamment non siliconés ou siliconés, ayant un paramètre de solubilité global selon l'espace de solubilité de Hansen inférieur ou égal à 18 $(MPa)^{1/2}$, de préférence inférieur ou égal à 17 $(MPa)^{1/2}$,
- les monoalcools ayant un paramètre de solubilité global selon l'espace de solubilité de Hansen inférieur ou égal à 20 $(MPa)^{1/2}$; et
- leurs mélanges.

  Le paramètre de solubilité global $\delta$ selon l'espace de solubilité de Hansen est défini dans l'article « Solubility parameter values » de Eric A.Grulke de l'ouvrage « Polymer Handbook », 3éme édition, Chapitre VII, p.519-559 par la relation :

$$\delta = (d_D^{\ 2} + d_P^{\ 2} + d_H^{\ 2})^{1/2}$$

dans laquelle

- $d_D$ caractérise les forces de dispersion de LONDON issues de la formation de dipôles induits lors des chocs moléculaires,
- $d_P$ caractérise les forces d'intéractions de DEBYE entre dipôles permanents, et
- $d_H$ caractérise les forces d'intéractions spécifiques (type liaisons hydrogène, acide/base, donneur/accepteur, etc.).

La définition des solvants dans l'espace de solubilité selon Hansen est décrite dans l'article de C.M.Hansen « The three dimensional solubility parameters » J.Paint Technol. 39, 105 (1967).

Parmi les composés liquides organiques, notamment non siliconés ou siliconés, ayant un paramètre de solubilité global selon l'espace de solubilité de Hansen inférieur ou égal à 18 $(Mpa)^{1/2}$, on peut citer des corps gras

liquides, notamment des huiles, qui peuvent être choisis parmi les huiles naturelles ou synthétiques, carbonées, hydrocarbonées, fluorées, siliconées, éventuellement ramifiées, seules ou en mélange.

Par "huile", on entend tout milieu non aqueux liquide à température ambiante (25°C) et pression atmosphérique (760mm de Hg), compatible avec une application sur la peau, les muqueuses (lèvres) et/ou les phanères (ongles, cils, sourcils, cheveux).

Parmi ces huiles, on peut citer les huiles végétales formées par des esters d'acides gras et de polyols, en particulier les triglycérides, telles que l'huile de tournesol, de sésame ou de colza, ou les esters dérivés d'acides ou d'alcools à longue chaîne (c'est-à-dire ayant de 6 à 20 atomes de carbone), notamment les esters de formule RCOOR' dans laquelle R représente le reste d'un acide gras supérieur comportant de 7 à 19 atomes de carbone et R' représente une chaîne hydrocarbonée comportant de 3 à 20 atomes de carbone, tels que les palmitates, les adipates et les benzoates, notamment l'adipate de diisopropyle.

On peut également citer les alcanes linéaires, ramifiés et/ou cycliques éventuellement volatils et notamment des huiles de paraffine, de vaseline, ou le polyisobutylène hydrogéné, l'isododécane, ou encore les 'ISOPARS', les isoparaffines volatiles. On peut citer également les esters, les éthers, les cétones. On peut encore citer les huiles siliconées telles que les polydiméthylsiloxanes et les polyméthylphénylsiloxanes, éventuellement substitués par des groupements aliphatiques et/ou aromatiques, éventuellement fluorés, ou par des groupements fonctionnels tels que des groupements hydroxyles, thiols et/ou amines, et les huiles siliconées volatiles, notamment cycliques.

En particulier, on peut citer les huiles de silicone, éventuellement ramifiées, volatiles et/ou non volatiles.

Par huile volatile, on entend tout milieu non aqueux susceptible de s'évaporer de la peau ou des lèvres, en moins d'une heure, ayant notamment une pression de vapeur, à température ambiante et pression atmosphérique allant de $10^{-3}$ à 300 mm de Hg (0,13 Pa à 40 000 Pa).

Comme huile siliconée volatile utilisable dans l'invention, on peut citer les silicones linéaires ou cycliques ayant de 2 à 7 atomes de silicium, ces silicones comportant éventuellement des groupes alkyle ou alkoxy ayant de 1 à 10 atomes de carbone. En particulier, on peut citer l'octaméthylcyclotétrasiloxane, le décaméthylcyclopentasiloxane, le dodécaméthylcyclohexasiloxane, l'heptaméthylhexyltrisiloxane, l'heptaméthyloctyltrisiloxane, l'octaméthyltrisiloxane, le décaméthyltétrasiloxane et leurs mélanges.

Comme huile siliconée non volatile, on peut citer les polydialkylsiloxanes non volatils, tels que les polydiméthylsiloxanes (PDMS) non volatils; les polydiméthylsiloxanes comportant des groupements alkyle, alcoxy ou phényle, pendant ou en bout de chaîne siliconée, groupements ayant de 2 à 24 atomes de carbone; les silicones phénylées comme les phényl triméthicones, les phényl diméthicones, les phényl triméthylsiloxy diphénylsiloxanes, les diphényl diméthicones, les diphényl méthyldiphényl trisiloxanes, les polyméthylphénylsiloxanes; les polysiloxanes modifiés par des acides gras (notamment en $C_8$-$C_{20}$), des alcools gras (notamment en $C_8$-$C_{20}$) ou des polyoxyalkylènes (notamment polyoxyéthyléne et/ou polyoxypropylène); les polysiloxanes aminées ; les polysiloxanes à groupement hydroxyles; les polysiloxanes fluorés comportant un groupement fluoré pendant ou en bout de chaîne siliconée ayant de 1 à 12 atomes de carbone dont tout ou partie des hydrogène sont substitués par des atomes de fluor ; et leurs mélanges.

On peut citer, en particulier, comme composés organiques liquides non siliconés ayant un paramètre de solubilité global selon l'espace de solubilité de Hansen inférieur ou égal à 18 $(Mpa)^{1/2}$ :

- les esters linéaires, ramifiés ou cycliques, ayant au moins 6 atomes de carbone, notamment de 6 à 30 atomes de carbone;
- les éthers ayant au moins 6 atomes de carbone, notamment de 6 à 30 atomes de carbone ; et
- les cétones ayant au moins 6 atomes de carbone, notamment de 6 à 30 atomes de carbone.

Par monoalcools liquides ayant un paramètre de solubilité global selon l'espace de solubilité de Hansen inférieur ou égal à 20 $(MPa)^{1/2}$, on entend les monoalcools liquides gras aliphatiques ayant de 6 à 30 atomes de carbone, la chaîne hydrocarbonée ne comportant pas de groupement de substitution. Comme monoalcools selon l'invention, on peut citer l'alcool oléique, le décanol et l'alcool linoléique.

Avantageusement, la composition selon l'invention peut comprendre une huile volatile en une teneur allant de 1 % à 90 % en poids, par rapport au poids total de la composition, et de préférence allant de 5 % à 70 % en poids.

La composition peut comprendre une huile non volatile en une teneur allant de 0,1 % à 80 % en poids, par rapport au poids total de la composition, et de préférence allant de 3 % à 50 % en poids.

Selon un premier mode de réalisation de l'invention, la phase grasse liquide peut être une phase grasse liquide non siliconée.

On entend par "phase grasse liquide non siliconée" une phase grasse comprenant un ou plusieurs composés organiques liquides ou huiles non siliconé(e)s, tels que ceux cités précédemment, lesdits composés non siliconés étant présents majoritairement dans la phase grasse liquide, c'est-à-dire à au moins 50 % en poids, notamment de 50 à 100 % en poids, de préférence de 60 % à 100 % en poids (par exemple de 60 à 99 % en poids), ou encore de 65 % à 100 % en poids (par exemple de 65 à 95 % en poids), par rapport au poids total de la phase grasse liquide.

Les composés organiques liquides non siliconés peuvent être notamment choisis parmi :

- les composés organiques liquides non siliconés ayant un paramètre de solubilité global selon l'espace de solubilité de Hansen inférieur ou égal à 18 $(MPa)^{1/2}$,
- les monoalcools ayant un paramètre de solubilité global selon l'espace de solubilité de Hansen inférieur ou égal à 20 $(MPa)^{1/2}$; et
- leurs mélanges.

Ladite phase grasse liquide non siliconée peut donc éventuellement comprendre des composés organiques liquide ou huiles siliconé(e)s, tels que ceux cités précédemment, qui peuvent être présents en une quantité inférieure à 50 % en poids, notamment allant de 0,1 à 40 % en poids, voire allant de 1 à 35 % en poids, ou encore allant de 5 à 30 % en poids, par rapport au poids total de la phase grasse liquide.
Selon un mode particulier de réalisation de l'invention, la phase grasse liquide non siliconée ne contient pas de composés organiques liquides ou huiles siliconé(e)s.
Lorsque la phase grasse liquide est une phase grasse liquide non siliconée, les macromonomères présents dans le polymère greffé sont avantageusement des macromonomères carbonés tels que décrits ci-après.
Lorsque la phase grasse liquide est une phase grasse liquide non siliconée, les macromonomères présents dans le polymère greffé sont avantageusement des macromonomères carbonés tels que décrits ci-après.
Par polymère greffé non siliconé, on entend un polymère greffé contenant majoritairement un macromonomère carboné et contenant éventuellement au plus 7 % en poids du poids total du polymère, de préférence au plus 5 % en poids, voire est exempt, de macromonomère siliconé.
Selon un deuxième mode de réalisation de l'invention, la phase grasse liquide peut être une phase grasse liquide siliconée.
On entend par "phase grasse liquide siliconée" une phase grasse comprenant un
ou plusieurs composés organiques liquides siliconés ou huiles siliconées tels que ceux décrits précédemment, lesdits composés siliconés étant présents majoritairement dans la phase grasse liquide, c'est-à-dire à au moins 50 % en poids, notamment de 50 à 100 % en poids, de préférence de 60 % à 100 % en poids (par exemple de 60 à 99 % en poids), ou encore de 65 % à 100 % en poids (par exemple de 65 à 95 % en poids), par rapport au poids total de la phase grasse liquide.
Les composés organiques liquides siliconés peuvent être notamment choisis parmi :

- les composés organiques liquides, notamment non siliconés ou siliconés, ayant un paramètre de solubilité global selon l'espace de solubilité de Hansen inférieur ou égal à 18 $(MPa)^{1/2}$.

Ladite phase grasse liquide siliconée peut donc éventuellement comprendre des composés organiques liquides ou huiles non siliconé(e)s, tels que décrits précédemment, qui peuvent être présents en une quantité inférieure à 50 % en poids, notamment allant de 0,1 à 40 % en poids, voire allant de 1 à 35 % en poids, ou encore allant de 5 à 30 % en poids, par rapport au poids total da la phase grasse liquide.
Selon un mode particulier de réalisation de l'invention, la phase grasse liquide siliconée ne contient pas de composés organiques liquides non siliconés.
Lorsque la phase grasse liquide est une phase grasse liquide siliconée, les macromonomères présents dans le polymère greffé sont avantageusement des macromonomères Lorsque la phase grasse liquide est une phase grasse liquide siliconée, les macromonomères présents dans le polymère greffé sont avantageusement des macromonomères siliconés tels que décrits ci-après.
En particulier, lorsque la phase grasse liquide est une phase grasse liquide siliconée, le polymère greffé présent dans la composition est avantageusement un polymère greffé siliconé.
Par polymère greffé siliconé, on entend un polymère greffé contenant majoritairement un macromonomère siliconé et contenant éventuellement au plus 7 % en poids du poids total du polymère, de préférence au plus 5 % en poids, voire est exempt, de macromonomère carboné. siliconés tels que décrits ci-après.
Le choix des monomères constituant le squelette du polymère, des macromonomères, le poids moléculaire du polymère, la proportion des monomères et des macromonomères peut être fait en fonction du milieu organique liquide de dispersion de manière à obtenir avantageusement une dispersion de particules de polymères greffés en particulier une dispersion stable, ce choix pouvant être effectué par l'homme du métier.
Par "dispersion stable", on entend une dispersion qui n'est pas susceptible de former de dépôt solide ou de déphasage liquide/solide notamment après une centrifugation, par exemple, à 4000 tours/minute pendant 15 minutes.
Le polymère éthylénique greffé formant les particules en dispersion comprend donc un squelette insoluble dans ledit milieu de dispersion et une partie soluble dans ledit milieu de dispersion.
Le polymère éthylénique greffé peut être un polymère statistique.

Selon l'invention, on entend par "polymère éthylénique greffé " un polymère susceptible d'être obtenu par polymérisation radicalaire :

- d'un ou plusieurs monomère(s) éthylénique(s),
- avec un ou plusieurs macromonomère(s), dans un milieu organique de polymérisation.

Selon l'invention, on entend par "polymère acrylique greffé " un polymère susceptible d'être obtenu par polymérisation radicalaire :

- d'un ou plusieurs monomère(s) acrylique(s), et éventuellement d'un ou plusieurs monomère(s) additionnel(s) vinylique(s) non acrylique(s),
- avec un ou plusieurs macromonomère(s), dans un milieu organique de polymérisation.

Avantageusement, les monomères acryliques représentent de 50 à 100 % en poids, de préférence de 55 à 100 % en poids (notamment de 55 à 95 % en poids), préférentiellement de 60 à 100 % en poids (notamment de 60 à 90 % en poids) du mélange monomères acryliques + monomères vinyliques non acryliques éventuels.

De préférence, les monomères acryliques sont choisis parmi les monomères dont l'homopolymère est insoluble dans le milieu de dispersion considéré, c'est-à-dire que l'homopolymère est sous forme solide (ou non dissous) à une concentration supérieure ou égale à 5% en poids à température ambiante (20°C) dans ledit milieu de dispersion.

Selon l'invention, on entend par "macromonomère ayant un groupe terminal polymérisable" tout polymère comportant sur une seule de ses extrémités un groupe terminal polymérisable apte à réagir lors de la réaction de polymérisation avec les monomères acryliques et éventuellement les monomères vinyliques non acryliques additionnels constituant le squelette. Le macromonomère permet de former les chaînes latérales du polymère acrylique greffé. Le groupe polymérisable du macromonomère peut être avantageusement un groupe à insaturation éthylénique susceptible de se polymériser par voie radicalaire avec les monomères constituant le squelette.

Par "macromonomère carboné" on entend un macromonomère non siliconé, et notamment un macromonomère oligomère obtenu par polymérisation de monomère(s) non siliconé(s) à insaturation éthylénique, et principalement par polymérisation de monomères acryliques et/ou vinyliques non acryliques.

Par "macromonomère siliconé" on entend un macromonomère organopolysiloxane, et en particulier un macromonomère polydiméthylsiloxane.

De préférence, le macromonomère est choisi parmi les macromonomères dont l'homopolymère est soluble dans le milieu de dispersion considéré, c'est-à-dire complètement dissous à une concentration supérieure ou égale à 5 % en poids et à température ambiante dans ledit milieu de dispersion.

Ainsi, le polymère acrylique greffé comprend un squelette (ou chaîne principale) constitué par un enchaînement de motifs acryliques résultant de la polymérisation notamment d'un ou plusieurs monomères acryliques et des chaînes latérales (ou greffons) issus de la réaction des macromonomères, lesdites chaînes latérales étant liées de manière covalente à ladite chaîne principale. Le squelette (ou chaîne principale) est insoluble dans le milieu de dispersion considéré alors que les chaînes latérales (ou greffons) sont solubles dans ledit milieu de dispersion.

Par "monomères acryliques", on entend dans la présente demande des monomères choisis parmi l'acide (méth)acrylique, les esters de l'acide (méth)acrylique (appelés également les (méth)acrylates), les amides de l'acide (méthacrylique) (appelés également les (méth)acrylamides).

Comme monomère acrylique susceptible d'être employé pour former le squelette insoluble du polymère, on peut citer, seul ou en mélange, les monomères suivants, ainsi que leurs sels :

- (i) les (méth)acrylates de formule :

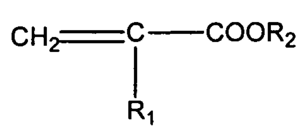

dans laquelle :

- $R_1$ désigne un atome d'hydrogène ou un groupe méthyle ;
- $R_2$ représente un groupe choisi parmi :

    - un groupe alkyle linéaire ou ramifié, comprenant de 1 à 6 atomes de carbone, ledit groupe pouvant comporter dans sa chaîne un ou plusieurs hétéroatomes choisis parmi O, N et S; et/ou pouvant comporter un ou plusieurs substituants choisis parmi -OH, les atomes d'halogène (F, Cl, Br, I) et - NR'R" avec R' et R" identiques ou différents choisis parmi les alkyles linéaires ou ramifiés en $C_1$-$C_4$; et/ou pouvant être substitué par au moins un groupe polyoxyalkylène, en particulier avec alkylène en $C_2$-$C_4$, notamment polyoxyéthylène et/ou polyoxypropylène, ledit groupe polyoxyalkylène étant constitué par la répétition de 5 à 30 motifs oxyalkylène;

- un groupe alkyle cyclique comprenant de 3 à 6 atomes de carbone, ledit groupe pouvant comporter dans sa chaîne un ou plusieurs hétéroatomes choisis parmi O, N et S, et/ou pouvant comporter un ou plusieurs substituants choisis parmi OH et les atomes d'halogène (F, Cl, Br, I);

A titre d'exemples de $R_2$, on peut citer le groupe méthyle, éthyle, propyle, butyle, isobutyle, méthoxyéthyle, éthoxyéthyle, méthoxy-polyoxyéthylène 350 OE , trifluoroéthyle, 2-hydroxyéthyle, 2-hydroxypropyle, diméthylaminoéthyle, diéthylaminoéthyle, diméthylaminopropyle.

- (ii) les (méth)acrylamides de formule :

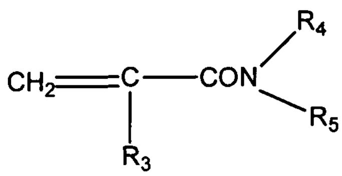

dans laquelle :

- $R_3$ désigne un atome d'hydrogène ou un groupe méthyle ;
- $R_4$ et $R_5$, identiques ou différents, représentent un atome d'hydrogène ou un groupe alkyle, linéaire ou ramifié, comportant de 1 à 6 atomes de carbone, pouvant comporter un ou plusieurs substituants choisis parmi -OH, les atomes d'halogène (F, Cl, Br, I) et -NR'R" avec R' et R" identiques ou différents choisis parmi les alkyles linéaires ou ramifiés en $C_1$-$C_4$;ou
- $R_4$ représente un atome d'hydrogène et $R_5$ représente un groupe 1,1-diméthyl-3-oxobutyle.
  A titre d'exemples de groupes alkyles pouvant constituer $R_4$ et $R_5$, on peut citer n-butyle, t-butyle, n-propyle, diméthylaminoéthyle, diéthylaminoéthyle, diméthylaminopropyle.

- (iii) les monomères (méth)acryliques comprenant au moins une fonction acide carboxylique, phosphorique ou sulfonique, tels que l'acide acrylique, l'acide méthacrylique, l'acide acrylamidopropanesulfonique.

Parmi ces monomères acryliques, on peut tout particulièrement citer les (méth)acrylates de méthyle, d'éthyle, de propyle, de butyle, d'isobutyle; les (méth)acrylates de méthoxyéthyle ou d'éthoxyéthyle; le méthacrylate de trifluoroéthyle; le méthacrylate de diméthylaminoéthyle, le méthacrylate de diéthylaminoéthyle, le méthacrylate de 2-hydroxypropyle, le méthacrylate de 2-hydroxyéthyle, l'acrylate de 2-hydroxypropyle, l'acrylate de 2-hydroxyéthyle ; le diméthylaminopropylméthacrylamide, l'acide (méth)acrylique ; et leurs sels ; et leurs mélanges.
De préférence, les monomères acryliques sont choisis parmi l'acrylate de méthyle, l'acrylate de méthoxyéthyle, le méthacrylate de méthyle, le méthacrylate de 2-hydroxyéthyle, l'acide (méth)acrylique, le méthacrylate de diméthylaminoéthyle, et leurs mélanges.
Parmi les monomères additionnels vinyliques non acryliques, on peut citer :

- les esters vinylique de formule : $R_6$-COO-CH=$CH_2$

dans laquelle $R_6$ représente un groupe alkyle linéaire ou ramifié, comprenant de 1 à 6 atomes, ou un groupe alkyle cyclique comportant de 3 à 6 atomes de carbone et/ou un groupe aromatique, par exemple de type benzénique, anthracénique, et naphtalénique ;

- les monomères vinyliques non acryliques comprenant au moins une fonction acide carboxylique, phosphorique ou sulfonique, tels que l'acide crotonique, l'anhydride maléique, l'acide itaconique, l'acide fumarique, l'acide maléique, l'acide styrènesulfonique, l'acide vinylbenzoïque, l'acide vinylphosphorique, et leurs sels ;
- les monomères vinyliques non acryliques comprenant au moins une fonction amine tertiaire, tels que la 2-vinylpyridine, la 4-vinylpyridine ;

- et leurs mélanges.

Avantageusement, les monomères acryliques présents dans le polymère greffé comprennent au moins un monomère choisi parmi les (méth)acrylates et les (méth)acrylamides décrits précédemment aux points (i) et (ii). De préférence, les monomères acryliques comprennent au moins un monomère choisi parmi les (méth)acrylates d'alkyle en $C_1$-$C_3$.
Le polymère greffé peut comprendre de l'acide (méth)acrylique.
Selon un mode de réalisation de l'invention, le polymère greffé ne comprend pas de monomère à fonction acide, notamment ne comprend pas d'acide (méth)acrylique.
Parmi les sels, on peut citer ceux obtenus par neutralisation des groupements acides à l'aide de base inorganiques telles que l'hydroxyde de sodium, l'hydroxyde de potassium, l'hydroxyde d'ammonium ou de bases organiques de type alcanols amines comme la monoéthanolamine, la diéthanolamine, la triéthanolamine, la 2-méthyl-2-amino-1-propanol.
On peut également citer les sels formés par neutralisation des motifs amine tertiaire, par exemple à l'aide d'acide minéral ou organique. Parmi les acides minéraux, on peut citer l'acide sulfurique ou l'acide chlorhydrique, l'acide bromhydrique, iodhydrique, l'acide phosphorique, l'acide borique. Parmi les acides organiques, on peut citer les acides comportant un ou plusieurs groupes carboxylique, sulfonique, ou phosphonique. Il peut s'agir d'acides aliphatiques linéaires, ramifiés ou cycliques ou encore d'acides aromatiques. Ces acides peuvent comporter, en outre, un ou plusieurs hétéroatomes choisis parmi O et N, par exemple sous la forme de groupes hydroxyles. On peut notamment citer l'acide acétique ou l'acide propionique, l'acide téréphtalique, ainsi que l'acide citrique et l'acide tartrique.
Selon un mode de réalisation de l'invention, le polymère éthylénique greffé ne contient pas de monomères vinyliques non acryliques additionnels tels que décrits précédemment. Dans ce mode de réalisation, le squelette insoluble du polymère éthylénique greffé est formé uniquement de monomères acryliques tels que décrits précédemment.
Il est entendu que ces monomères acryliques non polymérisés peuvent être solubles dans le milieu de dispersion considéré, mais le polymère formé avec ces monomères est insoluble dans le milieu de dispersion.
Selon un mode particulier de réalisation de l'invention, le polymère éthylénique greffé est susceptible d'être obtenu par polymérisation radicalaire dans un milieu organique de polymérisation :

- d'un monomère acrylique principal choisi parmi les (méth)acrylates d'alkyle en $C_1$-$C_3$, seul ou en mélange, et éventuellement d'un ou plusieurs monomères acryliques additionnels choisis parmi l'acide acrylique, l'acide méthacrylique et les (méth)acrylates d'alkyle de formule (I) définie ci-après, et leurs sels, pour former ledit squelette insoluble ; et
- et d'au moins un macromonomère siliconé comportant un groupe terminal polymérisable, tel que défini précédemment.

Comme monomère acrylique principal, on peut utiliser l'acrylate de méthyle, le méthacrylate de méthyle, l'acrylate d'éthyle, le méthacrylate d'éthyle, l'acrylate de n-propyle, le méthacrylate de n-propyle, l'acrylate d'iso-propyle et le méthacrylate d'iso-propyle, et leurs mélanges.
On préfère tout particulièrement l'acrylate de méthyle, le méthacrylate de méthyle, le méthacrylate d'éthyle.
Les monomères acryliques additionnels peuvent être choisis parmi :

- l'acide (méth)acrylique et ses sels,
- les (méth)acrylates de formule (I) et leurs sels :

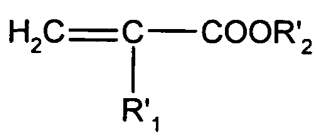

(I)

dans laquelle :

- $R'_1$ désigne un atome d'hydrogène ou un groupe méthyle ;
- $R'_2$ représente
    - un groupe alkyle linéaire ou ramifié comprenant de 1 à 6 atomes de carbone, ledit groupe comportant

dans sa chaîne un ou plusieurs atomes d'oxygène et/ou comportant un ou plusieurs substituants choisis parmi -OH, les atomes d'halogène (F, Cl, Br, I) et -NR'R" avec R' et R" identiques ou différents choisis parmi les alkyles linéaires ou ramifiés en $C_1$-$C_3$ ;

- un groupe alkyle cyclique comprenant de 3 à 6 atomes de carbone, ledit groupe pouvant comporter dans sa chaîne un ou plusieurs atomes d'oxygène et/ou pouvant comporter un ou plusieurs substituants choisis parmi OH et les atomes d'halogène (F, Cl, Br, I) ;

- et leurs mélanges.

A titre d'exemples de $R'_2$, on peut citer le groupe méthoxyéthyle, éthoxyéthyle, trifluoroéthyle; 2-hydroxyéthyle, 2-hydroxypropyle, diméthylaminoéthyle, diéthylaminoéthyle, diméthylaminopropyle.

Parmi ces monomères acryliques additionnels, on peut tout particulièrement citer l'acide (méth)acrylique, les (méth)acrylates de méthoxyéthyle ou d'éthoxyéthyle; le méthacrylate de trifluoroéthyle; le méthacrylate de diméthylaminoéthyle, le méthacrylate de diéthylaminoéthyle, le méthacrylate de 2-hydroxypropyle, le méthacrylate de 2-hydroxyéthyle, l'acrylate de 2-hydroxypropyle, l'acrylate de 2-hydroxyéthyle, leurs sels, et leurs mélanges.

Les macromonomères comportent à une des extrémités de la chaîne un groupe terminal polymérisable apte à réagir au cours de la polymérisation avec les monomères acryliques et éventuellement les monomères vinyliques additionnels, pour former les chaînes latérales du polymère éthylénique greffé. Ledit groupe terminal polymérisable peut être en particulier un groupe vinyle ou (méth)acrylate (ou (méth)acryl), et de préférence un groupe (méth)acrylate.

Les macromonomères sont choisis préférentiellement parmi les macromonomères dont l'homopolymère a une température de transition vitreuse (Tg) inférieure ou égale à 25°C, notamment allant de - 100°C à 25°C, de préférence allant de - 80°C à 0°C.

Les macromonomères ont une masse moléculaire moyenne en poids supérieure
ou égale à 200, de préférence supérieure ou égale à 300, préférentiellement supérieure ou égale à 500, et plus préférentiellement supérieure à 600.
De préférence, les macromonomères ont une masse moléculaire moyenne en poids (Mw) allant de 200 à 100 000, de préférence allant de 500 à 50 000, préférentiellement allant de 800 à 20 000, plus préférentiellement allant de 800 à 10000, et encore plus préférentiellement allant de 800 à 6000.

Dans la présente demande, les masses molaires moyennes en poids (Mw) et en nombre (Mn) sont déterminées par chromatographie liquide par perméation de gel (solvant THF, courbe d'étalonnage établie avec des étalons de polystyrène linéaire, détecteur réfractométrique).

Comme macromonomères carbonés, on peut en particulier citer :

- (i) les homopolymères et les copolymères (méth)acrylate d'alkyle linéaire ou ramifié en C8-C22, présentant un groupe terminal polymérisable choisi parmi les groupes vinyle ou (méth)acrylate parmi lesquels on peut citer en particulier : les macromonomères de poly(acrylate d'éthyl-2 hexyle) à extrémité mono(méth)acrylate ; les macromonomères de poly(acrylate de dodécyle) ou de poly(méthacrylate de dodécyle) à extrémité mono(méth)acrylate ; les macromonomères de poly(acrylate de stéaryle) ou de poly (méthacrylate de stéaryle) à extrémité mono(méth) acrylate.
  De tels macromonomères sont notamment décrits dans les brevets EP895467 et EP96459 et dans l'article Gillman K.F., Polymer Letters, Vol 5, page 477-481 (1967).
  On peut en particulier citer les macromonomères à base de poly(acrylate d'éthyl-2-hexyle) ou de poly(acrylate de dodécyle) à extrémité mono(méth)acrylate.

- (ii) les polyoléfines ayant un groupe terminal à insaturation éthylénique , en particulier ayant un groupement terminal (méth)acrylate. Comme exemple de telles polyoléfines, on peut citer en particulier les macromonomères suivants, étant entendu qu'ils ont un groupe terminal (méth)acrylate : les macromonomères de polyéthylène, les macromonomères de polypropylène, les macromonomères de copolymère polyéthylène/polypropylène, les macromonomères de copolymère polyéthylène/polybutylène, les macromonomères de polyisobutylène ; les macromonomères de polybutadiène; les macromonomères de polyisoprène ; les macromonomères de polybutadiène; les macromonomères de poly(éthylène/butylène)-polyisoprène ;
  De tels macromonomères sont en particulier décrits dans US5625005 qui mentionne des macromonomères éthylène/butylène et éthylène/propylène à groupement terminal réactif (méth)acrylate.

On peut en particulier citer le méthacrylate de poly(éthylène/butylène), tel que celui commercialisé sous la dénomination Kraton Liquid L-1253 par Kraton Polymers.

Comme macromonomères siliconés, on peut en particulier citer les polydiméthylsiloxanes à groupement terminal mono (méth)acrylate, et notamment ceux de formule (II) suivante :

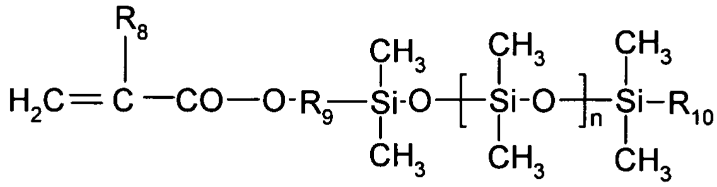

(II)

dans laquelle $R_8$ désigne un atome d'hydrogène ou un groupement méthyle ; Rg désigne un groupe hydrocarboné divalent ayant de 1 à 10 atomes de carbone et contient éventuellement une ou deux liaisons éther -O- ; R10 désigne un groupe alkyl ayant de 1 à 10 atomes de carbone, notamment de 2 à 8 atomes de carbone ; n désigne un nombre entier allant de 1 à 300, de préférence allant de 3 à 200, et préférentiellement allant de 5 à 100.

Comme macromonomères siliconés, on peut utiliser les monométhacryloxypropyl polydiméthylsiloxanes tels que ceux commercialisés sous la dénomination PS560-K6 par la société United Chemical Technologies Inc. (UCT) ou sous la dénomination MCR-M17 par la société Gelest Inc.

De préférence, le macromonomère polymérisé (constituant les chaînes latérales du polymère greffé) représente de 0,1 à 15 % en poids du poids total du polymère, préférentiellement de 0,2 à 10 % en poids, et plus préférentiellement de 0,3 à 8 % en poids.

Comme polymère éthylénique greffé particulièrement préféré dispersé dans une phase grasse liquide non siliconée, on peut utiliser ceux obtenus par polymérisation :

- de l'acrylate de méthyle et du macromonomère polyéthylène/polybutylène à groupement terminal méthacrylate (notamment Kraton L-1253), en particulier dans un solvant choisi parmi l'isododécane, l'isononanoate d'isononyle, l'octyldodécanol , le malate de diisostéaryle, un benzoate d'alkyl $C_{12}$-$C_{15}$ (tel que Finsolv TN).

Comme polymère acrylique greffé particulièrement préféré dispersé dans une phase grasse liquide siliconée, on peut utiliser celui obtenu par polymérisation :

- de l'acrylate de méthyle et du macromonomère monométhacryloxypropylpolydiméthylsiloxane ayant un poids moléculaire moyen en poids allant de 800 à 6000, en particulier dans le décaméthylcyclopentasiloxane ou le phényltriméthicone .

De préférence, le polymère greffé a une masse moléculaire moyenne en poids (Mw) comprise entre 10 000 et 300 000, notamment entre 20 000 et 200 000, mieux encore entre 25 000 et 150 000.

Grâce aux caractéristiques susmentionnées, dans un milieu organique de dispersion donné, les polymères ont la capacité de se replier sur eux-mêmes, formant ainsi des particules de forme sensiblement sphérique, avec sur le pourtour de ces particules les chaînes latérales déployées, qui assurent la stabilité de ces particules. De telles particules résultant des caractéristiques du polymère greffé ont la particularité de ne pas s'agglomérer dans ledit milieu et donc de s'autostabiliser et de former une dispersion de particules de polymère particulièrement stable.
En particulier, les polymères éthyléniques greffés de la dispersion peuvent former des particules nanométriques, de taille moyenne allant de 10 à 400 nm, de préférence de 20 à 200 nm.
Du fait de cette taille très faible, les particules de polymère greffé en dispersion sont particulièrement stables et donc peu susceptibles de former des agglomérats.
La dispersion de polymère greffé peut donc être une dispersion stable et ne forme pas de sédiments, lorsqu'elle est placée pendant une durée prolongée (par exemple 24 heures) à température ambiante (25 °C).

De préférence, la dispersion de particules de polymère greffé présente un taux de matière sèche (ou extrait sec) en polymère pouvant aller de 40 % à 70 % en poids de matière sèche, notamment allant de 45 % à 65 % en poids.

On peut préparer la dispersion de particules de polymère greffé par un procédé comprenant une étape de copolymérisation radicalaire, dans un milieu organique de polymérisation, d'un ou plusieurs monomères acryliques tels que définis précédemment avec un ou plusieurs macromonomères tels que définis précédemment.
Comme indiqué précédemment, le milieu organique liquide de dispersion peut être identique ou différent du milieu de polymérisation.

D'une manière classique, la copolymérisation peut être effectuée en présence d'un initiateur de polymérisation. Les initiateurs de polymérisation peuvent être des amorceurs radicalaires. De manière générale, un tel initiateur de polymérisation peut être choisi parmi les composés organiques peroxydés tels que le dilauroyl peroxyde, le dibenzoyl peroxyde, le tert-butyl peroxy-2-éthylhexanoate ; les composés diazotés tels que l'azobisisobutyronitrile, l'azobisdiméthylvalero-nitrile.

La réaction peut être également initiée à l'aide de photoinitiateurs ou par une radiation telle que des UV, des neutrons ou par plasma.
D'une manière générale, pour mettre en oeuvre ce procédé, on introduit, dans un réacteur de taille appropriée à la quantité de polymère que l'on va réaliser, au moins une partie du milieu organique de polymérisation, une partie des monomères acryliques et/ou vinyliques additionnels, qui constituera, après polymérisation, le squelette insoluble, la totalité du macromonomère (qui constituera les chaînes latérales du polymère) et une partie de l'initiateur de polymérisation. A ce stade d'introduction, le milieu réactionnel forme un milieu relativement homogène.
Le milieu réactionnel est ensuite agité et chauffé jusqu'à une température pour obtenir une polymérisation des monomères et macromonomères. Après un certain temps, le milieu initialement homogène et limpide conduit à une dispersion d'aspect laiteux. On ajoute ensuite un mélange constitué de la partie restante de monomères et de l'initiateur de polymérisation. Après un temps adéquat pendant lequel le mélange est chauffé sous agitation, le milieu se stabilise sous forme d'une dispersion laiteuse, la dispersion comprenant des particules de polymères stabilisés dans le milieu dans lequel elles ont été créées, ladite stabilisation étant due à la présence, dans le polymère, de chaînes latérales solubles dans ledit milieu de dispersion.

Avantageusement, le polymère greffé présent dans la composition selon l'invention présente au moins une température de transition vitreuse comprise entre 0 °C et 80 °C , de préférence comprise entre 0 °C et 60 °C, et préférentiellement comprise entre 0 °C et 40 °C. La température de transition vitreuse est notamment mesurée selon le protocole décrit précédemment relatif au dépôt obtenu avec la composition, par exemple en employant uniquement la dispersion de polymère greffé ; la température de transition vitreuse est ainsi mesurée sur un film ne contenant que le polymère greffé.

Le polymère greffé décrit précédemment peut être présent dans la composition selon l'invention en une teneur allant de 0,5 % à 45 % en poids, par rapport au poids total de la composition, de préférence allant de 1 % à 30 % en poids, et préférentiellement allant de 2 % à 25 % en poids.

Avantageusement, le polymère greffé est présent dans la fraction non volatile de la composition en une teneur supérieure ou égale à 20 % en poids de la dite fraction non volatile. On entend par fraction non volatile de la composition l'ensemble des constituants présents dans la composition qui ne sont pas volatiles. On entend par composé volatile un composé qui, pris isolément, a une pression de vapeur non nulle, à température ambiante (25 °C) et pression atmosphérique, allant en particulier de $10^{-3}$ à 300 mm de Hg (0,133 Pa à 40.000 Pa). De préférence, la teneur de polymère greffé dans la fraction non volatile de la composition peut aller de 20 % à 90 % en poids de ladite fraction non volatile, préférentiellement allant de 20 % à 70 % en poids, et plus préférentiellement allant de 20 % à 60 % en poids, et encore plus préférentiellement allant de 30 % à 60 % en poids.

La fraction non volatile de la composition correspond en fait au mélange des constituants restant sur la peau après le séchage complet de la composition appliquée sur la peau.

La composition selon l'invention peut comprendre une ou des matières colorantes choisies parmi les colorants hydrosolubles, et les matières colorantes pulvérulentes comme les pigments, les nacres, et les paillettes bien connues de l'homme du métier. Les matières colorantes peuvent être présentes, dans la composition, en une teneur allant de 0,01 % à 50 % en poids, par rapport au poids de la composition, de préférence de 0,01 % à 30 % en poids.

Par pigments, il faut comprendre des particules de toute forme, blanches ou colorées, minérales ou organiques, insolubles dans le milieu physiologique, destinées à colorer la composition.
Par nacres, il faut comprendre des particules de toute forme irisées, notamment produites par certains mollusques dans leur coquille ou bien synthétisées.

Les pigments peuvent être blancs ou colorés, minéraux et/ou organiques. On peut citer, parmi les pigments minéraux, le dioxyde de titane, éventuellement traité en surface, les oxydes de zirconium ou de cérium, ainsi que les oxydes de zinc, de fer (noir, jaune ou rouge) ou de chrome, le violet de manganèse, le bleu outremer, l'hydrate de chrome et le bleu ferrique, les poudres métalliques comme la poudre d'aluminium, la poudre de cuivre.
Parmi les pigments organiques, on peut citer le noir de carbone, les pigments de type D & C, et les laques à base de carmin de cochenille, de baryum, strontium, calcium, aluminium.

On peut également citer les pigments à effet tels les particules comportant un substrat organique ou minéral, naturel ou synthétique, par exemple le verre, les résines acrylique, le polyester, le polyuréthane, le polyéthylène téréphtalate, les céramiques ou les alumines, ledit substrat étant recouvert ou non de substances métalliques comme l'aluminium, l'or, l'argent, le platine, le cuivre, le bronze, ou d'oxydes métalliques comme le dioxyde de titane, l'oxyde de fer, l'oxyde de chrome et leurs mélanges.

Les pigments nacrés peuvent être choisis parmi les pigments nacrés blancs tels que le mica recouvert de titane, ou d'oxychlorure de bismuth, les pigments nacrés colorés tels que le mica titane recouvert avec des oxydes de fer, le mica titane recouvert avec notamment du bleu ferrique ou de l'oxyde de chrome, le mica titane recouvert avec un pigment organique du type précité ainsi que les pigments nacrés à base d'oxychlorure de bismuth.. On peut également utiliser les pigments interférentiels, notamment à cristaux liquides ou multicouches.

Avantageusement, le polymère greffé présent dans la composition selon l'invention permet d'obtenir une

bonne dispersion homogène des matières colorantes pulvérulents comme les pigments ou les nacres.

L'invention a donc également pour objet une composition de fond de teint comprenant une dispersion de particules de polymère éthylénique greffé dans une phase grasse liquide, telle que décrite précédemment, et au moins une matière colorante, notamment des pigments, des nacres, ou toute autre charge à effet optique.

Les colorants hydrosolubles sont par exemple le jus de betterave, le bleu de méthylène.

La composition selon l'invention peut comprendre en outre en outre une ou plusieurs charges, notamment en une teneur allant de 0,01 % à 50 % en poids, par rapport au poids total de la composition, de préférence allant de 0,01 % à 30 % en poids. Par charges, il faut comprendre des particules de toute forme, incolores ou blanches, minérales ou de synthèse, insolubles dans le milieu de la composition quelle que soit la température à laquelle la composition est fabriquée. Ces charges servent notamment à modifier la rhéologie ou la texture de la composition.

Les charges peuvent être minérales ou organiques de toute forme, plaquettaires, sphériques ou oblongues, quelle que soit la forme cristallographique (par exemple feuillet, cubique, hexagonale, orthorombique, etc). On peut citer le talc, le mica, la silice, le kaolin, les poudres de polyamide (Nylon®) (Orgasol® de chez Atochem), de poly-β-alanine et de polyéthylène, les poudres de polymères de tétrafluoroéthylène (Téflon®), la lauroyl-lysine, l'amidon, le nitrure de bore, les microsphères creuses polymériques telles que celles de chlorure de polyvinylidène/acrylonitrile comme l'Expancel® (Nobel Industrie), de copolymères d'acide acrylique (Polytrap® de la société Dow Corning) et les microbilles de résine de silicone (Tospearls® de Toshiba, par exemple), les particules de polyorganosiloxanes élastomères, le carbonate de calcium précipité, le carbonate et l'hydro-carbonate de magnésium, l'hydroxyapatite, les microsphères de silice creuses (Silica Beads® de Maprecos), les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium.

La composition selon l'invention peut également contenir des ingrédients couramment utilisés en cosmétique, tels que les vitamines, les agents hydratants, les émollients, les agents anti-radicaux libres, les épaississants, les oligo-éléments, les adoucissants, les séquestrants, les parfums, les agents alcalinisants
ou acidifiants, les conservateurs, les filtres solaires, les tensioactifs, les anti-oxydants, les gommes, les cires, les agents propulseurs, ou leurs mélanges.

Bien entendu, l'homme du métier veillera à choisir ce ou ces éventuels composés complémentaires, et/ou leur quantité, de manière telle que les propriétés avantageuses de la composition correspondante selon l'invention ne soient pas,
ou substantiellement pas, altérées par l'adjonction envisagée.

La composition selon l'invention peut se présenter notamment sous forme de suspension, de dispersion, de solution, de gel, d'émulsion, notamment émulsion huile-dans-eau (H/E) ou eau-dans-huile (E/H), ou multiple (E/H/E ou polyol/H/E ou H/E/H), sous forme de crème, de pâte, de mousse, de dispersion de vésicules notamment de lipides ioniques ou non, de lotion biphase ou multiphase, de spray, de poudre, de pâte. La composition peut être anhydre, par exemple il peut s'agir d'un stick ou d'une pâte anhydre. La composition peut être une composition non rinçée.

L'homme du métier pourra choisir la forme galénique appropriée, ainsi que sa méthode de préparation, sur la base de ses connaissances générales, en tenant compte d'une part de la nature des constituants utilisés, notamment de leur solubilité dans le support, et d'autre part de l'application envisagée pour la composition.

Selon un autre aspect, l'invention concerne également un ensemble cosmétique comprenant :

i) un récipient délimitant au moins un compartiment, ledit récipient étant fermé par un élément de fermeture ; et
ii) une composition disposée à l'intérieur dudit compartiment, la composition étant conforme à l'invention.

Le récipient peut être sous toute forme adéquate. Il peut être notamment sous forme d'un flacon, d'un tube, d'un pot, d'un étui, d'une boite, d'un sachet ou d'un boîtier.

L'élément de fermeture peut être sous forme d'un bouchon amovible, d'un couvercle, d'un opercule, d'une bande déchirable, ou d'une capsule, notamment du type comportant un corps fixé au récipient et une casquette articulée sur le corps. Il peut être également sous forme d'un élément assurant la fermeture sélective du récipient, notamment une pompe, une valve, ou un clapet.

Le récipient peut être associé à un applicateur, notamment sous forme d'un bloc de mousse ou d'élastomère, d'un feutre, ou d'une spatule. L'applicateur peut être libre (houppette ou éponge) ou solidaire d'une tige portée par l'élément de fermeture, tel que décrit par exemple dans le brevet US 5 492 426. L'applicateur peut être solidaire du récipient, tel que décrit par exemple le brevet FR 2 761 959.

Le produit peut être contenu directement dans le récipient, ou indirectement. A titre d'exemple, le produit peut être disposé sur un support imprégné, notamment sous forme d'une lingette ou d'un tampon, et disposé (à l'unité ou plusieurs) dans une boîte ou dans un sachet. Un tel support incorporant le produit est décrit par exemple dans la demande WO 01/03538.

L'élément de fermeture peut être couplé au récipient par vissage. Alternativement, le couplage entre l'élément

de fermeture et le récipient se fait autrement que par vissage, notamment via un mécanisme à baïonnette, par encliquetage, serrage, soudage, collage, ou par attraction magnétique. Par "encliquetage" on entend en particulier tout système impliquant le franchissement d'un bourrelet ou d'un cordon de matière par déformation élastique d'une portion, notamment de l'élément de fermeture, puis par retour en position non contrainte élastiquement de ladite portion après le franchissement du bourrelet ou du cordon.

Le récipient peut être au moins pour partie réalisé en matériau thermoplastique. A titre d'exemples de matériaux thermoplastiques, on peut citer le polypropylène ou le polyéthylène.

Alternativement, le récipient est réalisé en matériau non thermoplastique, notamment en verre ou en métal (ou alliage).

Le récipient peut être à parois rigides ou à parois déformables, notamment sous forme d'un tube ou d'un flacon tube.

Le récipient peut comprendre des moyens destinés à provoquer ou faciliter la distribution de la composition. A titre d'exemple, le récipient peut être à parois déformables de manière à provoquer la sortie de la composition en réponse à une surpression à l'intérieur du récipient, laquelle surpression est provoquée par écrasement élastique (ou non élastique) des parois du récipient. Alternativement, notamment lorsque le produit est sous forme d'un stick, ce dernier peut être entraîné par un mécanisme à piston. Toujours dans le cas d'un stick, notamment de produit de maquillage (rouge à lèvres, fond de teint, etc.), le récipient peut comporter un mécanisme, notamment à crémaillère, ou avec une tige filetée, ou avec une rampe hélicoïdale, et apte à déplacer un stick en direction de ladite ouverture. Un tel mécanisme est décrit par exemple dans le brevet FR 2 806 273

ou dans le brevet FR 2 775 566. Un tel mécanisme pour un produit liquide est décrit dans le brevet FR 2 727 609.

Le récipient peut être constitué d'un boîtier avec un fond délimitant au moins un logement contenant la composition, et un couvercle, notamment articulé sur le fond, et apte à recouvrir au moins en partie ledit fond. Un tel boîtier est décrit par exemple dans la demande WO 03/018423 ou dans le brevet FR 2 791 042.

Le récipient peut être équipé d'un essoreur disposé au voisinage de l'ouverture du récipient. Un tel essoreur permet d'essuyer l'applicateur et éventuellement, la tige dont il peut être solidaire. Un tel essoreur est décrit par exemple dans le brevet FR 2 792 618.

La composition peut être à la pression atmosphérique à l'intérieur du récipient (à température ambiante) ou pressurisée, notamment au moyen d'un gaz propulseur (aérosol). Dans ce dernier cas, le récipient est équipé d'une valve (du type de celles utilisées pour les aérosols).

Le contenu des brevets ou demandes de brevets cités précédemment sont incorporés par référence dans la présente demande.

L'invention va maintenant être décrite plus en détail à la lumière des exemples suivants donnés à titre illustratif et non limitatif.

Les présents exemples illustrent la préparation de polymères conformes à l'invention, aptes à former une dispersion de particules dans un milieu organique considéré.

Dans ces exemples, on détermine, après préparation de ladite dispersion, les masses molaires moyennes en poids (Mw) et en nombre (Mn) du polymère, la température de transition vitreuse du polymère, le taux de matière sèche (ou extrait sec) de la dispersion et la taille des particules de polymères.

Les masses molaires moyennes en poids (Mw) et en nombre (Mn) sont déterminées par chromatographie liquide par perméation de gel (solvant THF, courbe d'étalonnage établie avec des étalons de polystyrène linéaire, détecteur réfractométrique).

La mesure de la température de transition vitreuse (Tg) est effectuée selon la norme ASTM D3418-97, par analyse enthalpique différentielle (DSC "Differential Scanning Calorimetry") sur calorimètre, sur une plage de température comprise entre -100°C et +150°C à une vitesse de chauffe de 10°C/min dans des creusets en aluminium de 150 µl.

La préparation des creusets se fait de la manière suivante : dans un creuset en aluminium de 150 µl on introduit 100 µl de la dispersion obtenue et on laisse le solvant s'évaporer pendant 24h à température ambiante et à 50% d'humidité relative. On renouvelle l'opération puis on introduit le creuset dans le calorimètre Mettler DSC30.

Le taux de matière sèche (ou extrait sec), c'est à dire la teneur en matière non volatile, peut être mesurée de différentes manières : on peut citer par exemple les méthodes par séchage à l'étuve ou les méthodes par séchage par exposition à un rayonnement infrarouge.

De préférence, le taux de matière sèche est mesuré par échauffement de l'échantillon par des rayons infrarouges de 2 µm à 3,5 µm de longueur d'onde. Les substances contenues dans la composition qui possèdent une pression de vapeur élevée s'évaporent sous l'effet de ce rayonnement. La mesure de la perte de poids de l'échantillon permet de déterminer l'extrait sec de la composition. Ces mesures sont réalisées au moyen d'un dessiccateur à infrarouges commercial LP16 de chez Mettler. Cette technique est parfaitement décrite dans la documentation de l'appareil fournie par Mettler.

Le protocole de mesure est le suivant : on étale environ 1g de la composition sur une coupelle métallique. Celle-ci, après introduction dans le dessiccateur, est soumise à une consigne de température de 120°C pendant une heure.

La masse humide de l'échantillon, correspondant à la masse initiale et la masse sèche de l'échantillon, correspondant à la masse après exposition au rayonnement, sont mesurées au moyen d'une balance de précision.
Le taux de matière sèche est calculée de la manière suivante :

$$\text{Extrait Sec} = 100 \times (\text{masse sèche / masse humide}).$$

Les tailles de particules peuvent être mesurées par différentes techniques : on peut citer en particulier les techniques de diffusion de la lumière (dynamiques et statiques), les méthodes par compteur Coulter, les mesures par vitesse de sédimentation (reliée à la taille via la loi de Stokes) et la microscopie. Ces techniques permettent de mesurer un diamètre de particules et pour certaines d'entre elles une distribution granulométrique.
De préférence, les tailles et les distributions de tailles des particules des compositions selon l'invention, sont mesurées par diffusion statique de la lumière au moyen d'un granulomètre commercial de type MasterSizer 2000 de chez Malvern. Les données sont traitées sur la base de la théorie de diffusion de Mie. Cette théorie, exacte pour des particules isotropes, permet de déterminer dans le cas de particules non sphériques, un diamètre « effectif » de particules. Cette théorie est notamment décrite dans l'ouvrage de Van de Hulst, H.C., "Light Scattering by Small Particles," Chapitres 9 et 10, Wiley, New York, 1957. La composition est caractérisée par son diamètre « effectif » moyen en volume D[4,3], défini de la manière suivante :

$$D[4,3] = \frac{\sum_i V_i \cdot d_i}{\sum_i V_i}$$

où $V_i$ représente le volume des particules de diamètre effectif $d_i$. Ce paramètre est notamment décrit dans la documentation technique du granulomètre.
Les mesures sont réalisées à 25 °C, sur une dispersion de particules diluée, obtenue à partir de la composition de la manière suivante : 1) dilution d'un facteur 100 avec de l'eau, 2) homogénéisation de la solution, 3) repos de la solution durant 18 heures, 4) récupération du surnageant homogène blanchâtre.
Le diamètre « effectif » est obtenu en prenant un indice de réfraction de 1,33 pour l'eau et un indice de réfraction moyen de 1,42 pour les particules.
L'invention est illustrée plus en détails par les exemples décrits ci-après.

## Exemple 1 :

Préparation d'une dispersion de particules d'un polymère dans l'isododécane, ledit polymère étant obtenu par polymérisation d'acrylate de méthyle et le macromonomère correspondant à un copolymère polyéthylène/polybutylène à groupement terminal méthacrylate (Kraton L-1253).
Dans un réacteur, on charge 2 kg d'heptane, 2 kg d'isododécane, 2,8 kg d'acrylate de méthyle et 1,2 kg de macromonomère du type copolymère de polyéthylène/polybutylène à groupement terminal méthacrylate (Kraton L-1253) et 320 g de tertio butyl peroxy-2-éthylhexanoate (Trigonox 21 S).
On agite et on chauffe le mélange réactionnel à température ambiante à 90°C en 1 heure. Après 15 minutes à 90°C, on observe un changement d'aspect du milieu réactionnel, qui passe d'un aspect transparent à un aspect laiteux. On maintient le chauffage sous agitation pendant 15 minutes supplémentaires puis on ajoute goutte à goutte pendant 1 heure un mélange constitué par 16 kg d'acrylate de méthyle et 200 g de Trigonox 21 S.
On laisse ensuite le chauffage pendant 4 heures à 90°C puis on distille l'heptane du milieu réactionnel. A l'issue de cette opération de distillation, on obtient une dispersion de particules de polymère ainsi préparé stable dans l'isododécane
Le polymère greffé comprend 6% en poids de macromonomère par rapport au poids du polymère.
Les caractéristiques du polymère et des particules formées par ledit polymère sont les suivantes :

- Masse moléculaire poids Mw = 119900
- Masse moléculaire nombre Mn = 16300
- Indice de polydispersité (Mw/Mn) = 7.37
- Transition vitreuse : 10°C par DSC Mettler ;
- Extrait sec : 52.4 % dans l'isododécane, réalisé par thermobalance ;
- Granulométrie : 46 nm avec polydispersité de 0,05 réalisée sur Malvern Autosizer Lo-C à 25°C

**Exemple 2 :**

Cet exemple illustre la préparation d'un polymère formant une dispersion de particules dans une huile siliconée, ledit polymère étant obtenu par polymérisation d'acrylate de méthyle et du macromonomère monométhacryloxypropylpolydiméthylsiloxane ayant un poids moléculaire moyen en poids de 5000 vendu sous la dénomination MCR-M17 par la société Gelest Inc.

Dans un réacteur de 1 litre, on charge 200 g d'heptane, 200 g de décaméthylcyclopentasiloxane, 30 g d'acrylate de méthyle, 10 g de monométhacryloxypropylpolydiméthylsiloxane MCR-M17 et 3,2 g de tertiobutylperoxy-2-éthylhexanoate (Trigonox 21 S).

On agite et on chauffe le mélange réactionnel à 90°C en 1 heure. Après 15 minutes à 90°C, on observe un changement d'aspect du milieu réactionnel, qui passe d'un aspect transparent à un aspect laiteux. On maintient le chauffage sous agitation pendant 15 minutes supplémentaires puis on ajoute goutte à goutte pendant 1 heure un mélange constitué par 160 g d'acrylate de méthyle et 2 g de Trigonox 21 S. On laisse ensuite le chauffage pendant 4 heures à 90°C puis on distille l'heptane du milieu réactionnel.

A l'issue de cette opération de distillation, on obtient une dispersion de particules de polymère ainsi préparé stable dans le décaméthylcyclopentasiloxane (D5).

Le polymère greffé comprend 5 % en poids de macromonomère (donc de chaîne latérale soluble dans la D5) par rapport au poids du polymère.

Les caractéristiques du polymère et des particules formées par ledit polymère sont les suivantes :

- Masse moléculaire poids Mw=102347
- Masse moléculaire nombre Mn=28283
- Indice de polydispersité (Mw/Mn)= 3.62
- Extrait sec : 51,4 % dans le D5 réalisé par thermobalance
- Transition vitreuse : 12°C par DSC Mettler
- Granulométrie : 160 nm avec polydispersité de 0,04 réalisée sur Malvern Autosizer Lo-C à 25°C

Après mise en oeuvre du protocole de stabilité conformément à l'exemple 1, on constate que la dispersion est stable.

Les polymères des exemples 1 et 2 comprennent les monomères suivants , leurs taux étant indiqués en pourcentage en poids du polymère :

| Polymère | Ex 1 | Ex 2 |
|---|---|---|
| Acrylate de méthyle | 94 | 95 |
| Macromonomère carboné | 6 | - |
| Macromonomère siliconé | - | 5 |

**Exemple 3 :**

On a préparé un fond de teint ayant la composition suivante (les teneurs sont indiquées en pourcentage par rapport au poids total de la composition) :

| Exemple | 3 |
|---|---|
| Abil EM 97 | 0,9 |
| Inwitor 780 K | 0,3 |
| Isododécane | 37,4 |
| Dispersion de polymère greffé | Ex 1 26,8 |
| Kraton G 1701 E | 2,8 |
| D5 | 5 |
| Pigments | 12 |
| Poudre de nylon | 1,1 |

(suite)

| Exemple | 3 |
|---|---|
| Cire de polyéthylène | 1,4 |
| Parfum | 0,3 |
| Eau | 7 |
| Conservateurs | 1 |
| Octane-1,2-diol | 0,3 |
| Glycérine | 3 |
| Sulfate de magnésium | 0,7 |

La composition contient 15,2 % en poids de matière active de polymère greffé.
La teneur en polymère greffé dans la fraction non volatile de la composition est de 39 % en poids.

ingrédients utilisés :

ABIL EM 97 : mélange silicone oxyéthylénée oxypropylénée substituée en α-ω /cyclométhicone (85/15) vendu par la Société Goldschmidt

Inwitor 780 K : Mono-diglycerides d'acide iso-steraique estérifiés par de l'acide succinique, vendu par la Société Sasol

Kraton G1701 E : copolymère dibloc styrène/éthylène-propylène, vendu par la société Kraton Polymer

D5 : cyclopentadiméthylsiloxane

Poudre de nylon vendue sous la dénomination Orgasol® 2002 extra D NAT COS par la société ATOFINA
Cire de polyéthylène vendue sous la dénomination ceridust 9205F par la société Clariant
On a mesuré le module complexe E*, le pouvoir amortissant tgδ et la température de transition vitreuse du dépôt obtenu avec la composition selon le protocole de mesure décrit précédemment. On a obtenu les résultats suivants :

| Exemple | 3 |
|---|---|
| module complexe E* de conservation (MPa) | 34 |
| pouvoir amortissant tgδ | 1,3 |
| Tg (°C) | 26 |

De plus, ce fond de teint selon l'invention appliqué sur la peau permet d'obtenir un maquillage présentant de bonnes propriétés de confort, de non collant et de non transfert.

**Exemple 4 :**

On prépare une composition de fond de teint similaire à celle de l'exemple 3 en remplaçant la dispersion de polymère greffé de l'exemple 1 par celle de l'exemple 2.
On obtient ainsi une composition formant un film conforme à l'invention. Le fond de teint appliqué sur la peau conduit à un maquillage de la peau confortable, non collant, et présentant de bonnes propriétés non transfert.

## Revendications

1. Composition cosmétique de maquillage ou de soin de la peau comprenant une dispersion de particules d'un polymère éthylénique greffé dans une phase grasse liquide, la composition étant apte à former un dépôt ayant un module complexe E* de conservation inférieur ou égal à 200 MPa.

**2.** Composition selon la revendication 1, **caractérisée par le fait que** le dépôt a un module complexe E* de conservation inférieur ou égal à 150 MPa, de préférence inférieur ou égal à 100 MPa, et préférentiellement inférieur ou égal à 50 MPa.

**3.** Composition selon la revendication 1 ou 2, **caractérisée par le fait que** la composition est apte à former un dépôt ayant un pouvoir amortissant tgδ supérieur ou égal à 0,7 , de préférence supérieur ou égal à 0,9, préférentiellement supérieur ou égal à 1,1.

**4.** Composition selon l'une quelconque des revendications précédentes, **caractérisée par le fait que** la composition est apte à former un dépôt ayant au moins une température de transition vitreuse comprise entre 0 et 40 °C.

**5.** Composition selon l'une quelconque des revendications précédentes, **caractérisée par le fait que** la composition comprend une fraction non volatile et que le polymère éthylénique greffé est présent en une teneur supérieure ou égale à 20 % en poids par rapport au poids de ladite fraction non volatile.

**6.** Composition selon la revendication précédente, **caractérisée par le fait que** le polymère éthylénique greffé est présent en une teneur allant de 20 % à 90 % en poids, par rapport au poids total de ladite fraction non volatile de la composition.

**7.** Composition selon l'une quelconque des revendications 8 et 9, **caractérisée par le fait que** le polymère éthylénique greffé est présent en une teneur allant de 20 % à 70 % en poids, et plus préférentiellement allant de 20 % à 60 % en poids, par rapport au poids total de ladite fraction non volatile de la composition.

**8.** Composition selon l'une quelconque des revendications précédentes, **caractérisée par le fait que** le polymère éthylénique greffé comprend un squelette éthylénique insoluble dans ladite phase grasse liquide et des chaînes latérales liées de manière covalente audit squelette et solubles dans ladite phase grasse liquide.

**9.** Composition selon l'une quelconque des revendications précédentes, **caractérisée par le fait que** le polymère éthylénique greffé est un polymère acrylique greffé.

**10.** Composition selon l'une quelconque des revendications précédentes, **caractérisée par le fait que** le polymère éthylénique est dispersé en l'absence de stabilisant additionnel en surface des particules du polymère greffé.

**11.** Composition selon l'une quelconque des revendications précédentes, **caractérisée par le fait que** le polymère éthylénique greffé en dispersion est un polymère acrylique susceptible d'être obtenu par polymérisation radicalaire dans un milieu organique de polymérisation :

- d'au moins un monomère acrylique, et éventuellement d'au moins un monomère additionnel vinylique non acrylique, pour former ledit squelette insoluble ; et
- d'au moins un macromonomère comportant un groupe terminal polymérisable pour former les chaînes latérales, ledit macromonomère ayant une masse moléculaire moyenne en poids supérieure ou égale à 200 et la teneur en macromonomère polymérisé représentant de 0,05 à 20% en poids du polymère.

**12.** Composition selon la revendication précédente, **caractérisée par le fait que** le monomère acrylique est choisi parmi, seul ou en mélange, les monomères suivants, ainsi que leurs sels :

- (i) les (méth)acrylates de formule :

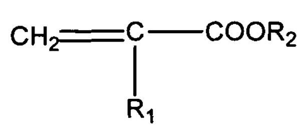

dans laquelle :

- $R_1$ désigne un atome d'hydrogène ou un groupe méthyle ;
- $R_2$ représente un groupe choisi parmi :

  - un groupe alkyle linéaire ou ramifié, comprenant de 1 à 6 atomes de carbone, ledit groupe pouvant comporter dans sa chaîne un ou plusieurs hétéroatomes choisis parmi O, N et S; et/ou pouvant comporter un ou plusieurs substituants choisis parmi -OH, les atomes d'halogène (F, Cl, Br, I) et -NR'R" avec R' et R" identiques ou différents choisis parmi les alkyles linéaires ou ramifiés en $C_1$-$C_4$; et/ou pouvant être substitué par au moins un groupe polyoxyalkylène, notamment polyoxyéthylène et/ou polyoxypropylène, ledit groupe polyoxyalkylène étant constitué par la répétition de 5 à 30 motifs oxyalkylène ;
  - un groupe alkyle cyclique comprenant de 3 à 6 atomes de carbone, ledit groupe pouvant comporter dans sa chaîne un ou plusieurs hétéroatomes choisis parmi O, N et S, et/ou pouvant comporter un ou plusieurs substituants choisis parmi OH et les atomes d'halogène (F, Cl, Br, I) ;

- (ii) les (méth)acrylamides de formule :

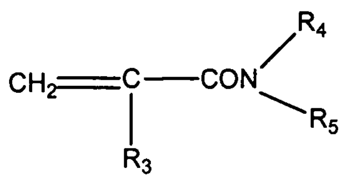

dans laquelle :

- $R_3$ désigne un atome d'hydrogène ou un groupe méthyle ;
- $R_4$ et $R_5$, identiques ou différents, représentent un atome d'hydrogène ou un groupe alkyle, linéaire ou ramifié, comportant de 1 à 6 atomes de carbone, pouvant comporter un ou plusieurs substituants choisis parmi -OH, les atomes d'halogène (F, Cl, Br, I) et -NR'R" avec R' et R" identiques ou différents choisis parmi les alkyles linéaires ou ramifiés en $C_1$-$C_4$;ou
- $R_4$ représente un atome d'hydrogène et $R_5$ représente un groupe 1,1-diméthyl-3-oxobutyle ;

- (iii) les monomères (méth)acryliques comprenant au moins une fonction acide carboxylique, phosphorique ou sulfonique, tels que l'acide acrylique, l'acide méthacrylique, l'acide acrylamidopropanesulfonique.

**13.** Composition selon la revendication 11 ou 12, **caractérisée par le fait que** le monomère acrylique est choisi parmi les (méth)acrylates de méthyle, d'éthyle, de propyle, de butyle, d'isobutyle; les (méth)acrylates de méthoxyéthyle ou d'éthoxyéthyle ; le méthacrylate de trifluoroéthyle; le méthacrylate de diméthylaminoéthyle, le méthacrylate de diéthylaminoéthyle, le (méth)acrylate de 2-hydroxypropyle, le (méth)acrylate de 2-hydroxyéthyle ; le diméthylaminopropylméthacrylamide; l'acide (méth)acrylique ; et leurs sels.

**14.** Composition selon l'une quelconque des revendications 11 à 13, **caractérisée par le fait que** le monomère acrylique est choisi parmi l'acrylate de méthyle, l'acrylate de méthoxyéthyle, le méthacrylate de méthyle, le méthacrylate de 2-hydroxyéthyle, l'acide (méth)acrylique, le méthacrylate de diméthylaminoéthyle, et leurs mélanges.

**15.** Composition selon l'une quelconque des revendications 11 à 14, **caractérisée par le fait que** le polymère greffé comprend de l'acide (méth)acrylique.

**16.** Composition selon l'une quelconque des revendications 11 à 14, **caractérisée par le fait que** les monomères acryliques comprennent au moins un monomère choisi parmi les (méth)acrylates et les (méth)acrylamides décrits aux points (i) et (ii) dans la revendication 8.

**17.** Composition selon l'une quelconque des revendications 11 à 16, **caractérisée par le fait que** les monomères acryliques comprennent au moins un monomère choisi parmi les (méth)acrylates d'alkyle en $C_1$-$C_3$.

**18.** Composition selon l'une quelconque des revendications 11 à 17, **caractérisée par le fait que** le polymère acrylique greffé ne contient pas de monomère à fonction acide.

**19.** Composition selon l'une quelconque des revendications 11 à 18, **caractérisée par le fait que** le polymère acrylique greffé ne contient pas de monomère vinylique non acrylique additionnel.

**20.** Composition selon l'une quelconque des revendications 11 à 18, **caractérisée par le fait que** le polymère acrylique greffé est susceptible d'être obtenu par polymérisation radicalaire d'un ou plusieurs monomère(s) acrylique(s) et d'un un ou plusieurs monomère(s) additionnel(s) vinylique(s) non acrylique(s), et dudit macromonomère.

**21.** Composition selon l'une quelconque des revendications 11 à 18 et 20, **caractérisée par le fait que** les monomères additionnels vinyliques non acryliques sont choisis parmi :

- les esters vinylique de formule : $R_6$-COO-CH=$CH_2$

dans laquelle $R_6$ représente un groupe alkyle linéaire ou ramifié, comprenant de 1 à 6 atomes, ou un groupe alkyle cyclique comportant de 3 à 6 atomes de carbone et/ou un groupe aromatique, par exemple de type benzénique, anthracénique, et naphtalénique ;

- les monomères vinyliques non acryliques comprenant au moins une fonction acide carboxylique, phosphorique ou sulfonique, tels que l'acide crotonique, l'anhydride maléique, l'acide itaconique, l'acide fumarique, l'acide maléique, l'acide styrènesulfonique, l'acide vinylbenzoïque, l'acide vinylphosphorique et les sels de ceux-ci ;
- les monomères vinyliques non acryliques comprenant au moins une fonction amine tertiaire, tels que la 2-vinylpyridine, la 4-vinylpyridine ;
- et leurs mélanges.

**22.** Composition selon l'une quelconque des revendications 11 à 21, **caractérisée par le fait que** les monomères acryliques représentent de 50 à 100 % en poids, de préférence de 60 à 100 % en poids , préférentiellement de 70 à 100 % en poids du mélange monomères acryliques + monomères vinyliques non acryliques éventuels.

**23.** Composition selon l'une quelconque des revendications 11 à 22, **caractérisée par le fait que** le macromonomère comporte à une des extrémités de la chaîne un groupe terminal polymérisable choisi parmi un groupe vinyle ou un groupe (méth)acrylate, et de préférence un groupe (méth)acrylate.

**24.** Composition selon l'une quelconque des revendications 11 à 23, **caractérisée par le fait que** le macromonomère a une masse moléculaire moyenne en poids supérieure ou égale à 300, préférentiellement supérieure ou égale à 500, et plus préférentiellement supérieure à 600.

**25.** Composition selon l'une quelconque des revendications 11 à 24, **caractérisée par le fait que** le macromonomère a une masse moléculaire moyenne en poids (Mw) allant de 200 à 100 000, de préférence allant de 500 à 50 000, préférentiellement allant de 800 à 20 000, plus préférentiellement allant de 800 à 10000, et encore plus préférentiellement allant de 800 à 6000.

**26.** Composition selon l'une quelconque des revendications 11 à 25, **caractérisée par le fait que** le macromonomère polymérisé représente de 0,1 à 15 % en poids du poids total du polymère, de préférence de 0,2 à 10 % en poids, et préférentiellement de 0,3 à 8 % en poids.

**27.** Composition selon l'une quelconque des revendications précédentes, **caractérisée par le fait que** la phase grasse liquide comprend un composé organique liquide choisi parmi :

- les composés organiques liquides ayant un paramètre de solubilité global selon l'espace de solubilité de Hansen inférieur ou égal à 18 $(MPa)^{1/2}$,
- les monoalcools ayant un paramètre de solubilité global selon l'espace de solubilité de Hansen inférieur ou égal à 20 $(MPa)^{1/2}$; et
- leurs mélanges.

**28.** Composition selon la revendication précédente, **caractérisée par le fait que** la ledit composé organique liquide est une huile non volatile.

**29.** Composition selon l'une quelconque des revendications précédentes, **caractérisée par le fait que** la phase grasse liquide est une phase grasse liquide non siliconée.

**30.** Composition selon la revendication précédente, **caractérisée par le fait que** la phase grasse liquide non siliconée est constituée d'au moins 50% en poids d'au moins un composé liquide organique non siliconé choisi parmi :

- les composés liquides organiques non siliconés ayant un paramètre de solubilité global selon l'espace de solubilité de Hansen inférieur ou égal à 18 $(MPa)^{1/2}$ ;
- les monoalcools liquides ayant un paramètre de solubilité global selon l'espace de solubilité de Hansen inférieur ou égal à 20 $(MPa)^{1/2}$ ; et
- leurs mélanges.

**31.** Composition selon la revendication 29 ou 30, **caractérisée par le fait que** la phase grasse liquide non siliconée comprend moins de 50 % en poids de composés organiques liquides siliconés ayant un paramètre de solubilité global selon l'espace de solubilité de Hansen inférieur ou égal à 18 $(MPa)^{1/2}$.

**32.** Composition selon la revendication 29 ou 30, **caractérisée par le fait que** la phase grasse liquide non siliconée ne contient pas de composés organiques liquides siliconés.

**33.** Composition selon l'une quelconque des revendications 29 à 32, **caractérisée par le fait que** le macromonomère est un macromonomère carboné.

**34.** Composition selon la revendication précédente, **caractérisée par le fait que** le macromonomère carboné est choisi parmi :

- (i) les homopolymères et les copolymères d'acrylate ou de méthacrylate d'alkyle en $C_8$-$C_{22}$ linéaire ou ramifié et ayant un groupe terminal polymérisable choisi parmi les groupes vinyle ou (méth)acrylate ;
- (ii) les polyoléfines ayant un groupe terminal à insaturation éthylénique polymérisable.

**35.** Composition selon la revendication 33 ou 34, **caractérisée par le fait que** le macromonomère carboné est choisi parmi :

- (i) les macromonomères de poly(acrylate d'éthyl-2 hexyle) à extrémité mono(méth)acrylate ; les macromonomères de poly(acrylate de dodécyle) à extrémité mono(méth)acrylate ; les macromonomères de poly(méthacrylate de dodécyle) ; les macromonomères de poly(acrylate de stéaryle) à extrémité mono(méth)acrylate ; les macromonomères de poly (méthacrylate de stéaryle) à extrémité mono(méth)acrylate ;

- (ii) les macromonomères de polyéthylène, les macromonomères de polypropylène, les macromonomères de copolymère polyéthylène/polypropylène, les macromonomères de copolymère polyéthylène/polybutylène, les macromonomères de polyisobutylène, les macromonomères de polybutadiène, les macromonomères de polyisoprène, les macromonomères de polybutadiène, les macromonomères de poly(éthylène/butylène)-polyisoprène, ces macromonomères ayant un groupement terminal (méth)acrylate.

**36.** Composition selon l'une quelconque des revendications 33 à 35, **caractérisée par le fait que** le macromonomère carboné est choisi parmi :

- (i) les macromonomères de poly(acrylate d'éthyl-2-hexyle) à extrémité mono(méth)acrylate, les macromonomères de poly(acrylate de dodécyle) à extrémité mono(méth)acrylate ;
- (ii) le méthacrylate de poly(éthylène/butylène).

**37.** Composition selon l'une quelconque des revendications 33 à 36, **caractérisée par le fait que** le polymère greffé est obtenu par polymérisation :

- de l'acrylate de méthyle et du macromonomère polyéthylène/polybutylène à groupement terminal méthacrylate, en particulier dans un solvant choisi parmi l'isododécane, l'isononylisononanoate, l'octyldodécanol , le malate de diisostéaryle, un benzoate d'alkyl $C_{12}$-$C_{15}$ .

**38.** Composition selon l'une quelconque des revendications 29 à 37, **caractérisée par le fait que** le polymère greffé est un polymère greffé non siliconé.

**39.** Composition selon la revendication précédente, **caractérisée par le fait que** le polymère greffé non siliconé contient majoritairement un macromonomère carboné et contenant éventuellement au plus 7 % en poids, du poids total du polymère, de macromonomère siliconé.

**40.** Composition selon la revendication 38 ou 40, **caractérisée par le fait que** le polymère greffé siliconé est exempt de macromonomère carboné.

**41.** Composition selon l'une quelconque des revendications 1 à 28, **caractérisée par le fait que** la phase grasse liquide est une phase grasse liquide siliconée.

**42.** Composition selon la revendication précédente, **caractérisée par le fait que** la phase grasse liquide siliconée est constituée d'au moins 50 % en poids d'au moins un composé liquide organique siliconé choisi parmi les composés liquides organiques siliconés ayant un paramètre de solubilité global selon l'espace de solubilité de Hansen inférieur ou égal à 18 $(MPa)^{1/2}$.

**43.** Composition selon la revendication 31 ou 42, **caractérisée par** le fait le composé liquide organique siliconé comprend une huile siliconée volatile.

**44.** Composition selon la revendication précédente, **caractérisée par le fait que** l'huile siliconée volatile est choisie parmi l'octaméthylcyclotétrasiloxane, le décaméthylcyclopentasiloxane, le dodécaméthylcyclohexasiloxane, l'heptaméthylhexyltrisiloxane, l'heptaméthyloctyltrisiloxane, l'octaméthyltrisiloxane, le décaméthyltétrasiloxane, et leurs mélanges.

**45.** Composition selon la revendication 31 ou 42, **caractérisée par** le fait le composé liquide organique siliconé comprend une huile siliconée non volatile.

**46.** Composition selon la revendication précédente, **caractérisée par le fait que** l'huile siliconée non volatile est choisie parmi les polydialkylsiloxanes non volatils; les polydiméthylsiloxanes comportant des groupements alkyle, alcoxy ou phényle, pendant ou en bout de chaîne siliconée, groupements ayant de 2 à 24 atomes de carbone; les silicones phénylées ; les polysiloxanes modifiés par des acides gras (notamment en $C_8$-$C_{20}$), des alcools gras (notamment en $C_8$-$C_{20}$) ou des polyoxyalkylènes (notamment polyoxyéthyléne et/ou polyoxypropylène); les polysiloxanes aminées ; les polysiloxanes à groupement hydroxyles; les polysiloxanes fluorés comportant un groupement fluoré pendant ou en bout de chaîne siliconée ayant de 1 à 12 atomes de carbone dont tout ou partie des hydrogène sont substitués par des atomes de fluor ; et leurs mélanges.

**47.** Composition selon l'une quelconque des revendications 41 à 46, **caractérisée par le fait que** la phase grasse liquide comprend moins de 50 % en poids de composés organiques liquides non siliconés.

**48.** Composition selon la revendication 30 ou 47, **caractérisée par le fait que** le composé organique liquide non siliconé est choisi parmi les composés organiques liquides non siliconés ayant un paramètre de solubilité global selon l'espace de solubilité de Hansen inférieur à 18 $(MPa)^{1/2}$ ; les monoalcools liquides ayant un paramètre de solubilité global selon l'espace de solubilité de Hansen inférieur ou égal à 20 $(MPa)^{1/2}$ ; et leurs mélanges.

**49.** Composition selon la revendication précédente, **caractérisée par le fait que** le composé liquide organique non siliconé ayant un paramètre de solubilité global selon l'espace de solubilité de Hansen inférieur ou égal à 18 $(MPa)^{1/2}$ est choisi parmi les huiles carbonées, hydrocarbonées, fluorées, seules ou en mélange ; les alcanes linéaires, ramifiés et/ou cycliques, éventuellement volatils; les esters et notamment les esters linéaires, ramifiés ou cycliques, ayant au moins 6 atomes de carbone; les cétones et notamment les cétones ayant au moins 6 atomes de carbone; les éthers et notamment les éthers ayant au moins 6 atomes de carbone.

**50.** Composition selon la revendication 27 ou 48, **caractérisée par le fait que** les monoalcools ayant un paramètre de solubilité global selon l'espace de solubilité de Hansen inférieur ou égal à 20 $(MPa)^{1/2}$ sont choisis parmi les monoalcools gras aliphatiques ayant 6 à 30 atomes de carbone, la chaîne hydrocarbonée ne comportant pas de groupement de substitution, et notamment l'alcool oléique, le décanol, et l'alcool linoléique.

**51.** Composition selon l'une quelconque des revendications précédentes, **caractérisée par le fait que** la phase grasse liquide comprend une huile volatile non siliconée.

**52.** Composition selon la revendication précédente, **caractérisée par le fait que** l'huile volatile non siliconée est choisie parmi l'isododécane, l'isodécane, l'isohexadécane.

**53.** Composition selon l'une quelconque des revendications 41 à 50, **caractérisée par le fait que** la phase grasse liquide ne contient pas de composés organiques liquides non siliconés.

**54.** Composition selon l'une quelconque des revendications précédentes, **caractérisée par le fait que** l'huile non volatile est présente en une teneur allant de 0,1 % à 80 % en poids, par rapport au poids total de la composition, et de préférence allant de 3 % à 50 % en poids.

**55.** Composition selon l'une quelconque des revendications précédentes, **caractérisée par le fait qu'**elle comprend une huile volatile en une teneur allant de 1 % à 90 % en poids, par rapport au poids total de la composition, et de préférence allant de 5 % à 70 % en poids.

**56.** Composition selon l'une quelconque des revendications 41 à 55, **caractérisée par le fait que** le macromonomère est un macromonomère siliconé.

**57.** Composition selon la revendication précédente, **caractérisée par le fait que** le macromonomère siliconé est un macromonomère organopolysiloxane, et de préférence un macromonomère polydiméthylsiloxane.

**58.** Composition selon la revendication 56 ou 57, **caractérisée par le fait que** le macromonomère est choisi parmi les polydiméthylsiloxanes à groupement terminal mono (méth)acrylate, et notamment les monométhacryloxypropyl polydiméthylsiloxanes.

**59.** Composition selon l'une quelconque des revendications 56 à 58, **caractérisée par le fait que** le macromonomère siliconé est choisi parmi les macromonomères de formule (II) suivante :

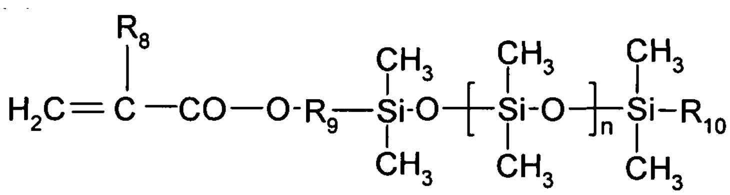

(II)

dans laquelle $R_8$ désigne un atome d'hydrogène ou un groupement méthyle ; Rg désigne un groupe hydrocarboné divalent ayant de 1 à 10 atomes de carbone et contient éventuellement une ou deux liaisons éther -O- ; R10 désigne un groupe alkyl ayant de 1 à 10 atomes de carbone, notamment de 2 à 8 atomes de carbone ; n désigne un nombre entier allant de 1 à 300, de préférence allant de 3 à 200, et préférentiellement allant de 5 à 100.

**60.** Composition selon l'une quelconque des revendications 41 à 59, **caractérisée par le fait que** le polymère acrylique greffé est susceptible d'être obtenu par polymérisation radicalaire dans le milieu de polymérisation :

- d'un monomère acrylique principal choisi parmi les (méth)acrylates d'alkyle en $C_1$-$C_3$, seul ou en mélange, et éventuellement d'un ou plusieurs monomères acryliques additionnels choisis parmi l'acide acrylique, l'acide méthacrylique et les (méth)acrylates d'alkyle de formule (I) :

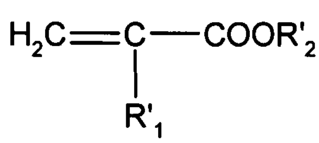

(I)

dans laquelle :

- $R'_1$ désigne un atome d'hydrogène ou un groupe méthyle ;
- $R'_2$ représente

- un groupe alkyle linéaire ou ramifié comprenant de 1 à 6 atomes de carbone, ledit groupe comportant dans sa chaîne un ou plusieurs atomes d'oxygène et/ou comportant un ou plusieurs substituants choisis parmi
  -OH, les atomes d'halogène (F, Cl, Br, I) et -NR'R" avec R' et R", identiques ou différents, choisis parmi les alkyles linéaires ou ramifiés en $C_1$-$C_3$ ;
- un groupe alkyle cyclique comprenant de 3 à 6 atomes de carbone, ledit groupe pouvant comporter dans sa chaîne un ou plusieurs atomes d'oxygène et/ou pouvant comporter un ou plusieurs substituants choisis parmi OH et les atomes d'halogène (F, Cl, Br, I) ;

et leurs sels, pour former ledit squelette insoluble ; et

- et d'un macromonomère siliconé.

**61.** Composition selon la revendication précédente, **caractérisée par le fait que** $R'_2$ désigne un groupe choisi parmi les groupes méthoxyéthyle, éthoxyéthyle, trifluoroéthyle; 2-hydroxyéthyle, 2-hydroxypropyle, diméthylaminoéthyle, diéthylaminoéthyle, diméthylaminopropyle.

**62.** Composition selon la revendication 60 ou 61, **caractérisée par le fait que** le monomère acrylique principal est choisi parmi le (méth)acrylate de méthyle, le (méth)acrylate d'éthyle, le (méth)acrylate de n-propyle, le (méth) acrylate d'iso-propyle, et leurs mélanges.

**63.** Composition selon l'une quelconque des revendications 60 à 62, **caractérisée par le fait que** le monomère acrylique principal est choisi parmi l'acrylate de méthyle, le méthacrylate de méthyle, le méthacrylate d'éthyle.

**64.** Composition selon la revendication 60, **caractérisée par le fait que** le monomère acrylique additionnel est choisi parmi l'acide (méth)acrylique, le (méth)acrylate de méthoxyéthyle, le (méth)acrylate d'éthoxyéthyle, le méthacrylate de trifluoroéthyle, le méthacrylate de diméthylaminoéthyle, le méthacrylate de diéthylaminoéthyle, le (méth) acrylate de 2-hydroxypropyle, le (méth)acrylate de 2-hydroxyéthyle, et leurs sels.

**65.** Composition selon la revendication 60 ou 64, **caractérisée par le fait que** le monomère acrylique additionnel est choisi parmi l'acide acrylique, l'acide méthacrylique.

**66.** Composition selon l'une quelconque des revendications 41 à 65, **caractérisée par le fait que** le polymère greffé est choisi parmi les polymères obtenus par polymérisation :

- de l'acrylate de méthyle et du macromonomère monométhacryloxypropylpolydiméthylsiloxane ayant un poids moléculaire moyen en poids allant de 800 à 6000, en particulier dans le décaméthylcyclopentasiloxane ou le phényltriméthicone .

**67.** Composition selon l'une quelconque des revendications 41 à 66, **caractérisée par le fait que** le polymère greffé est un polymère greffé siliconé.

**68.** Composition selon la revendication précédente, **caractérisée par le fait que** le polymère greffé siliconé contient majoritairement un macromonomère siliconé et contenant éventuellement au plus 7 % en poids, du poids total du polymère, de macromonomère carboné.

**69.** Composition selon la revendication 67 ou 68, **caractérisée par le fait que** le polymère greffé siliconé est exempt de macromonomère carboné.

**70.** Composition selon l'une quelconque des revendications précédentes, **caractérisée par le fait que** le polymère greffé a une masse moléculaire moyenne en poids (Mw) comprise entre 10 000 et 300 000, notamment entre 20 000 et 200 000, mieux encore entre 25 000 et 150 000.

**71.** Composition selon l'une quelconque des revendications précédentes, **caractérisée par le fait que** les particules de polymère greffé ont une taille moyenne allant de 10 à 400 nm, de préférence allant de 20 à 200 nm.

**72.** Composition selon l'une quelconque des revendications précédentes, **caractérisée par le fait que** le polymère éthylénique greffé est un polymère filmogène.

**73.** Composition selon l'une quelconque des revendications précédentes, **caractérisée par le fait que** le polymère éthylénique greffé à au moins une température de transition vitreuse comprise entre 0 ° C et 80 °C, de préférence entre 0 °C et 60 °C, et préférentiellement comprise entre 0 °C et 40 °C.

**74.** Composition selon l'une quelconque des revendications précédentes, **caractérisée par le fait que** le polymère greffé est présent en une teneur allant de 0,5 % à 45 % en poids, par rapport au poids total de la composition, de préférence allant de 1 % à 30 % en poids, et préférentiellement allant de 2 % à 25 % en poids.

**75.** Composition selon l'une quelconque des revendications précédentes, **caractérisée par le fait qu'**elle comprend une matière colorante.

**76.** Composition selon la revendication précédente, **caractérisée par le fait que** la matière colorante est une matière colorante pulvérulente, notamment choisie parmi les pigments et les nacres.

**77.** Composition selon l'une quelconque des revendications précédentes, **caractérisée par le fait qu'**elle comprend un ingrédient cosmétique choisi parmi les les vitamines, les agents hydratants, les émollients, les agents anti-radicaux libres, les épaississants, les oligo-éléments, les adoucissants, les séquestrants, les parfums, les agents alcalinisants ou acidifiants, les conservateurs, les filtres solaires, les tensioactifs, les anti-oxydants, les gommes, les cires, les agents propulseurs, ou leurs mélanges.

**78.** Composition cosmétique selon l'une quelconque des revendications précédentes, **caractérisée par le fait qu'**elle se présente sous forme de suspension, de dispersion, de solution, de gel, d'émulsion, notamment émulsion huile-dans-eau (H/E) ou eau-dans-huile (E/H), ou multiple (E/H/E ou polyol/H/E ou H/E/H), sous forme de crème, de pâte, de mousse, de dispersion de vésicules notamment de lipides ioniques ou non, de lotion biphase ou multiphase, de spray, de poudre.

**79.** Composition cosmétique selon l'une quelconque des revendications précédentes, **caractérisée par le fait qu'**elle se présente sous forme anhydre.

**80.** Composition selon l'une quelconque des revendications précédentes, **caractérisée par le fait que** la composition est un produit de maquillage de la peau.

**81.** Fond de teint comprenant une composition selon l'une quelconque des revendications précédentes.

**82.** Composition de fond de teint comprenant une dispersion de particules d'un polymère éthylénique greffé dans une phase grasse liquide telle que définie selon l'une des revendications 1 à 74, et une matière colorante, notamment des pigments, des nacres ou toute autre charge à effet optique.

**83.** Ensemble cosmétique comprenant :

a) un récipient délimitant au moins un compartiment, ledit récipient étant fermé par un élément de fermeture ; et
b) une composition disposée à l'intérieur dudit compartiment, la composition étant conforme à l'une quelconque des revendications qui précèdent.

**84.** Ensemble cosmétique selon la revendication précédente, **caractérisé par le fait que** le récipient est formé, au moins pour partie, en au moins un matériau thermoplastique.

**85.** Ensemble cosmétique selon la revendication 83, **caractérisé par le fait que** le récipient est formé, au moins pour partie, en au moins un matériau non thermoplastique, notamment en verre ou en métal.

**86.** Ensemble selon l'une quelconque des revendications 83 à 85, **caractérisé par le fait que**, en position fermée du récipient, l'élément de fermeture est vissé sur le récipient.

**87.** Ensemble selon l'une quelconque des revendications 3 à 85, **caractérisé par le fait que**, en position fermée du récipient, l'élément de fermeture est couplé au récipient autrement que par vissage, notamment par encliquetage, collage, ou soudage.

**88.** Ensemble selon l'une quelconque des revendications 83 à 87, **caractérisé par le fait que** la composition est

sensiblement à la pression atmosphérique à l'intérieur du compartiment.

**89.** Ensemble selon l'une quelconque des revendications 83 à 88, **caractérisé par le fait que** la composition est pressurisée à l'intérieur du récipient.

**90.** Procédé cosmétique de maquillage ou de soin de la peau comprenant l'application sur la peau d'une composition cosmétique selon l'une des revendications 1 à 82.

**91.** Utilisation d'une composition selon l'une des revendications 1 à 82, pour obtenir un dépôt, notamment un maquillage, sur la peau ayant une bonne adhérence et/ou confortable et/ou ayant une bonne résistance au transfert, notamment en présence de sébum.

**92.** Utilisation, dans une composition cosmétique, d'une dispersion de particules d'un polymère éthylénique greffé en dispersion dans une phase grasse liquide, la composition étant notamment apte à former un dépôt ayant un module complexe E* inférieur ou égal à 200 MPa, pour obtenir un dépôt, notamment un maquillage, sur la peau ayant une bonne adhérence et/ou confortable et/ou ayant une bonne résistance au transfert, notamment en présence de sébum.

Office européen des brevets

# RAPPORT DE RECHERCHE EUROPEENNE

Numéro de la demande
EP 05 29 1072

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| E | WO 2005/058274 A (L'OREAL ET AL.) 30 juin 2005 (2005-06-30) * revendications 1,3-13,15-68,86,115,119-122,128-134,136 * * page 9, ligne 8 - ligne 12 * * page 35, ligne 15 - ligne 21 * * page 81, ligne 25 - ligne 28 * ----- | 1-92 | A61K7/48 |
| X | EP 0 987 012 A (L'OREAL) 22 mars 2000 (2000-03-22) | 1-4, 27-32, 41-55, 67-82, 90-92 | |
| Y | * le document en entier * ----- | 5-26, 33-40, 56-65, 83-89 | |
| Y | EP 0 815 848 A (L'OREAL) 7 janvier 1998 (1998-01-07) * le document en entier * ----- | 5-26, 33-40, 60-65 | |
| Y | EP 0 687 462 A (L'OREAL) 20 décembre 1995 (1995-12-20) * le document en entier * ----- | 56-60 | |
| Y | EP 1 421 928 A (L'OREAL) 26 mai 2004 (2004-05-26) * revendications 72-76 * ----- | 83-88 | |
| Y | EP 1 245 506 A (L'OREAL) 2 octobre 2002 (2002-10-02) * alinéas [0001], [0002] * ----- | 89 | |

-/--

DOMAINES TECHNIQUES RECHERCHES (Int.Cl.7)
A61K

Le présent rapport a été établi pour toutes les revendications

| Lieu de la recherche | Date d'achèvement de la recherche | Examinateur |
|---|---|---|
| Berlin | 7 septembre 2005 | Alvarez Alvarez, C |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

1

Office européen des brevets

# RAPPORT DE RECHERCHE EUROPEENNE

Numéro de la demande
EP 05 29 1072

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| A,D | EP 0 749 747 A (L'OREAL) 27 décembre 1996 (1996-12-27) * le document en entier * ----- | 1-92 | |
| A | EP 1 380 603 A (L'OREAL) 14 janvier 2004 (2004-01-14) * alinéas [0022], [0023]; revendications 6-9 * ----- | 1-3 | |

DOMAINES TECHNIQUES RECHERCHES (Int.Cl.7)

Le présent rapport a été établi pour toutes les revendications

1

| Lieu de la recherche | Date d'achèvement de la recherche | Examinateur |
|---|---|---|
| Berlin | 7 septembre 2005 | Alvarez Alvarez, C |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
.......................................................................
& : membre de la même famille, document correspondant

EPO FORM 1503 03.82 (P04C02)

## ANNEXE AU RAPPORT DE RECHERCHE EUROPEENNE RELATIF A LA DEMANDE DE BREVET EUROPEEN NO.

EP 05 29 1072

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

07-09-2005

| Document brevet cité au rapport de recherche | | Date de publication | Membre(s) de la famille de brevet(s) | | Date de publication |
|---|---|---|---|---|---|
| WO 2005058274 | A | 30-06-2005 | AU | 2003301500 A1 | 09-07-2004 |
| | | | FR | 2863493 A1 | 17-06-2005 |
| | | | WO | 2005058274 A1 | 30-06-2005 |
| | | | AU | 2003300601 A1 | 09-07-2004 |
| | | | AU | 2003300602 A1 | 09-07-2004 |
| EP 0987012 | A | 22-03-2000 | FR | 2783415 A1 | 24-03-2000 |
| | | | BR | 9905295 A | 20-03-2001 |
| | | | CA | 2281992 A1 | 18-03-2000 |
| | | | CN | 1249171 A | 05-04-2000 |
| | | | EP | 0987012 A1 | 22-03-2000 |
| | | | JP | 2000119124 A | 25-04-2000 |
| | | | KR | 2000023142 A | 25-04-2000 |
| | | | US | 6326013 B1 | 04-12-2001 |
| EP 0815848 | A | 07-01-1998 | FR | 2750321 A1 | 02-01-1998 |
| | | | AT | 189118 T | 15-02-2000 |
| | | | CA | 2209462 A1 | 28-12-1997 |
| | | | DE | 69701206 D1 | 02-03-2000 |
| | | | DE | 69701206 T2 | 08-06-2000 |
| | | | EP | 0815848 A1 | 07-01-1998 |
| | | | ES | 2144832 T3 | 16-06-2000 |
| | | | JP | 3212540 B2 | 25-09-2001 |
| | | | JP | 10059816 A | 03-03-1998 |
| | | | JP | 3616032 B2 | 02-02-2005 |
| | | | JP | 2001354516 A | 25-12-2001 |
| | | | PT | 815848 T | 31-07-2000 |
| | | | US | 6113882 A | 05-09-2000 |
| EP 0687462 | A | 20-12-1995 | FR | 2721034 A1 | 15-12-1995 |
| | | | AT | 144136 T | 15-11-1996 |
| | | | CA | 2151189 A1 | 09-12-1995 |
| | | | DE | 69500069 D1 | 21-11-1996 |
| | | | DE | 69500069 T2 | 20-02-1997 |
| | | | EP | 0687462 A1 | 20-12-1995 |
| | | | ES | 2095784 T3 | 16-02-1997 |
| | | | JP | 2587801 B2 | 05-03-1997 |
| | | | JP | 7330539 A | 19-12-1995 |
| | | | US | 5650159 A | 22-07-1997 |
| EP 1421928 | A | 26-05-2004 | AU | 2003283477 A1 | 19-04-2004 |
| | | | AU | 2003283478 A1 | 19-04-2004 |
| | | | AU | 2003285389 A1 | 19-04-2004 |
| | | | AU | 2003286204 A1 | 19-04-2004 |
| | | | AU | 2003288305 A1 | 19-04-2004 |
| | | | AU | 2003290143 A1 | 19-04-2004 |

EPO FORM P0460

Pour tout renseignement concernant cette annexe : voir Journal Officiel de l'Office européen des brevets, No.12/82

**ANNEXE AU RAPPORT DE RECHERCHE EUROPEENNE RELATIF A LA DEMANDE DE BREVET EUROPEEN NO.** EP 05 29 1072

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

07-09-2005

| Document brevet cité au rapport de recherche | Date de publication | Membre(s) de la famille de brevet(s) | Date de publication |
|---|---|---|---|
| EP 1421928 A | | AU 2003299069 A1 | 19-04-2004 |
| | | AU 2003299070 A1 | 19-04-2004 |
| | | AU 2003299071 A1 | 19-04-2004 |
| | | AU 2003299072 A1 | 19-04-2004 |
| | | BR 0303890 A | 08-09-2004 |
| | | BR 0303891 A | 08-09-2004 |
| | | BR 0314489 A | 02-08-2005 |
| | | CN 1504488 A | 16-06-2004 |
| | | EP 1411069 A2 | 21-04-2004 |
| | | EP 1421928 A2 | 26-05-2004 |
| | | EP 1545450 A2 | 29-06-2005 |
| | | EP 1545438 A2 | 29-06-2005 |
| | | EP 1545443 A2 | 29-06-2005 |
| | | EP 1545439 A2 | 29-06-2005 |
| | | EP 1545440 A2 | 29-06-2005 |
| | | EP 1565148 A2 | 24-08-2005 |
| | | EP 1545441 A2 | 29-06-2005 |
| | | EP 1545436 A2 | 29-06-2005 |
| | | EP 1545442 A2 | 29-06-2005 |
| | | WO 2004028492 A2 | 08-04-2004 |
| | | WO 2004028493 A2 | 08-04-2004 |
| | | WO 2004028488 A2 | 08-04-2004 |
| | | WO 2004028489 A2 | 08-04-2004 |
| | | WO 2004028490 A2 | 08-04-2004 |
| | | WO 2004028491 A2 | 08-04-2004 |
| | | WO 2004028494 A2 | 08-04-2004 |
| | | WO 2004028485 A2 | 08-04-2004 |
| | | WO 2004028486 A2 | 08-04-2004 |
| | | WO 2004028487 A2 | 08-04-2004 |
| | | JP 2004149772 A | 27-05-2004 |
| | | JP 2004269497 A | 30-09-2004 |
| | | US 2004120906 A1 | 24-06-2004 |
| | | US 2004120920 A1 | 24-06-2004 |
| EP 1245506 A | 02-10-2002 | FR 2822655 A1 | 04-10-2002 |
| | | CA 2378612 A1 | 27-09-2002 |
| | | EP 1245506 A1 | 02-10-2002 |
| | | JP 2003002376 A | 08-01-2003 |
| | | US 2002145005 A1 | 10-10-2002 |
| EP 0749747 A | 27-12-1996 | FR 2735689 A1 | 27-12-1996 |
| | | AT 174502 T | 15-01-1999 |
| | | CA 2197496 A1 | 09-01-1997 |
| | | DE 69601147 D1 | 28-01-1999 |
| | | DE 69601147 T2 | 02-06-1999 |
| | | EP 0749747 A1 | 27-12-1996 |

EPO FORM P0460

Pour tout renseignement concernant cette annexe : voir Journal Officiel de l'Office européen des brevets, No.12/82

**ANNEXE AU RAPPORT DE RECHERCHE EUROPEENNE RELATIF A LA DEMANDE DE BREVET EUROPEEN NO.** EP 05 29 1072

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

07-09-2005

| Document brevet cité au rapport de recherche | Date de publication | Membre(s) de la famille de brevet(s) | Date de publication |
|---|---|---|---|
| EP 0749747 A | | ES 2128149 T3 | 01-05-1999 |
| | | WO 9700663 A1 | 09-01-1997 |
| | | JP 2000044426 A | 15-02-2000 |
| | | JP 3225967 B2 | 05-11-2001 |
| | | JP 10501005 T | 27-01-1998 |
| | | US 5851517 A | 22-12-1998 |
| EP 1380603 A | 14-01-2004 | FR 2841774 A1 | 09-01-2004 |
| | | EP 1380603 A1 | 14-01-2004 |
| | | JP 2004035561 A | 05-02-2004 |
| | | US 2004071644 A1 | 15-04-2004 |

EPO FORM P0460

Pour tout renseignement concernant cette annexe : voir Journal Officiel de l'Office européen des brevets, No.12/82